# NEUE EUROPÄISCHE PATENTSCHRIFT

(11) **EP 3 539 665 B2**
(45) Veröffentlichungstag und Bekanntmachung des Hinweises auf die Entscheidung über den Einspruch: **18.12.2024**
(45) Hinweis auf die Patenterteilung: 03.08.2022
(21) Anmeldenummer: 18163436.1
(22) Anmeldetag: 22.03.2018
(51) Int. Cl.: B01L 3/02, G05B 15/00

(54) **ELEKTRONISCHES LABOR-DOSIERSYSTEM FÜR FLÜSSIGKEITEN UND VERFAHREN ZUM BETRIEB EINES ELEKTRONISCHEN LABOR-DOSIERSYSTEMS FÜR FLÜSSIGKEITEN**
ELECTRONIC LABORATORY DOSING SYSTEM FOR LIQUIDS AND METHOD FOR OPERATING AN ELECTRONIC LABORATORY DOSING SYSTEM FOR LIQUIDS
SYSTÈME DE DOSAGE DE LABORATOIRE ÉLECTRONIQUE POUR LIQUIDES ET PROCÉDÉ DE FONCTIONNEMENT D'UN SYSTÈME DE DOSAGE DE LABORATOIRE ÉLECTRONIQUE POUR LIQUIDES

(30) Priorität: 16.03.2018 EP 18162322
(43) Veröffentlichungstag der Anmeldung: 18.09.2019
(73) Patentinhaber: Eppendorf SE, 22339 Hamburg (DE)
(72) Erfinder: HACKER, Jan-Hendrik, 22393 Hamburg (DE); MOLITOR, Peter, 22143 Hamburg (DE)
(74) Vertreter: Patent- und Rechtsanwälte Behrmann Wagner PartG mbB

(56) Entgegenhaltungen:
- EP-A1- 1 452 849
- EP-A1- 3 189 896
- US-A1- 2004 171 171
- US-A1- 2007 056 351
- US-B2- 7 770 475

## Beschreibung

Die vorliegende Erfindung bezieht sich auf ein elektronisches Labor-Dosiersystem für Flüssigkeiten gemäß dem Oberbegriff des Anspruchs 1.

Ferner betrifft die Erfindung ein Verfahren zum Betrieb eines elektronischen Labor-Dosiersystems für Flüssigkeiten gemäß dem Oberbegriff des Anspruchs 10

Aus dem Stand der Technik sind Labor-Dosiersysteme und Verfahren zu deren Betrieb bereits bekannt. Beispielsweise offenbart die EP 0 999 432 B1 ein Verfahren zum Betreiben eines elektronischen Dosiersystems und ein Dosiersystem, bei dem über eine Schnittstelle zwischen einer Handdosiervorrichtung und einer Datenverarbeitungsanlage Daten übertragbar sind, die gewährleisten, dass Routinen für den Betrieb der Handdosiervorrichtung auf der Datenverarbeitungsanlage oder einer der Datenverarbeitungsanlage zugeordneten Eingabeschnittstelle definiert und an die Handdosiervorrichtung übermittelt und seitens der Handdosiervorrichtung gespeichert werden können. Dies ermöglicht einerseits eine einfache Bedienung oder Programmierung der Handdosiervorrichtung zur Vorbereitung und Durchführung von komplexen Arbeitsroutinen. Andererseits ermöglicht ein derartiges System sowie das entsprechende Verfahren bereits eine gewisse Anleitung eines Bedieners bei dem Betrieb oder im Rahmen der Durchführung von entsprechenden Arbeitsroutinen.

Aus der EP 3 141 909 A1 ist außerdem bereits ein Labor-Dosiersystem für Flüssigkeiten bekannt, bei dem ein optischer Vergleich anhand einer Kamera durchgeführt wird und einem Bediener anhand des optischen Vergleichs eine Ausgabe vermittelt oder angezeigt wird, die auf mögliche Fehler oder Ungenauigkeiten im Rahmen der Durchführung von Arbeitsroutinen hinweisen soll. Dadurch kann grundsätzlich die Einhaltung der vorgegebenen Routinen während der Durchführung der Arbeitsroutinen verbessert bzw. Fehler durch einen Bediener während des Bedienens der Handdosiervorrichtung leichter kenntlich gemacht werden.

Außerdem sind aus dem Stand der Technik bereits Systeme und Verfahren bekannt, die bei einem Einsatz zum Betrieb eines elektronischen Labor-Dosiersystems eine Protokollierung der durchgeführten Arbeitsroutinen anhand von entsprechenden Betriebsdaten der Handdosiervorrichtung und/oder anhand von dem Betrieb der Handdosiervorrichtung zumindest mittelbar erfassenden und/oder Betriebsumweltbedingungen erfassende Kontextparametersensoreinheiten ermöglicht.

Beispielsweise ist aus der EP 1 452 849 A1 bereits bekannt Daten betreffend die Herstellung einer Lösung, insbesondere vorausberechnete Umrechnungsergebnisse zwischen Volumen und Masse, mittels eines an eine Waage angeschlossenen Druckers auszudrucken.

Nachteilig am Stand der Technik ist jedoch, dass die bisher bekannten Verfahren und Systeme die Anforderungen an ein Qualitäts-Management der mit dem System hergestellten Proben und/oder Flüssigkeiten nur unzureichend oder nicht erfüllt. Denn für die jeweils hergestellten oder bereitgestellten Flüssigkeiten ist seitens des Systems oder durch das Verfahren nur eine äußerst lückenhafte Nachverfolgung der Proben oder Flüssigkeiten möglich. Dies bedeutet, dass insbesondere im Nachhinein die korrekte und vollständige Durchführung oder Abarbeitung der jeweiligen Arbeitsroutine zur Herstellung der Probe oder der Flüssigkeit nicht oder nur rudimentär nachvollzogen werden kann.

Ausgehend von diesem Stand der Technik liegt der Erfindung daher die Aufgabe zugrunde, ein elektronisches Labor-Dosiersystem für Flüssigkeiten anzugeben, welches eine selbstständige oder automatische Validierung von durchgeführten oder abgearbeiteten Arbeitsroutinen ermöglicht und damit die Nachverfolgbarkeit von hergestellten Flüssigkeiten verbessert und darüber das Qualitätsmanagement für Labore und vergleichbare Einrichtungen erleichtert und verbessert.

Ferner besteht die Aufgabe darin, ein Verfahren anzugeben, welches ein verbessertes Qualitäts-Management in Laboren und vergleichbaren Einrichtungen erlaubt.

Dieser Aufgabe wird hinsichtlich des Systems mit den Merkmalen des Anspruchs 1 gelöst. Hinsichtlich des Verfahrens wird die Aufgabe mit den Merkmalen des unabhängigen Anspruchs 10 gelöst.

Vorteilhafte Weiterbildungen der Erfindung sind in den Unteransprüchen angegeben. Den Rahmen der Erfindung bilden sämtliche Kombinationen aus zumindest zwei von in der Beschreibung, den Ansprüchen und/oder den Figuren offenbarten Merkmalen.

Der Erfindung liegt demnach der Gedanke zugrunde, dass im Rahmen des Betriebs der Handdosiervorrichtung, also bei Arbeitsroutinen jeglicher Art, einschließlich Routinen zur Kalibrierung der Handdosiervorrichtung, einerseits möglichst umfassende und aussagekräftige Daten über den Betrieb selbst erfasst und protokolliert werden, was durch die Betriebssensoreinheit und die Kontextparametersensoreinheit erreicht wird, wobei zusätzlich zu dem reinen Erfassen von Daten über den Betrieb und die Umweltbedingungen des Betriebs zusätzlich Vorgaben oder Solldaten geschaffen und vorgehalten werden, die einen optimalen Betrieb oder eine korrekte Handhabung sowie korrekte Umweltbedingungen beschreiben, und dass schließlich im Rahmen des Betriebs der Handdosiervorrichtung oder aber im Anschluss an den Betrieb der Handdosiervorrichtung ein Vergleich der Betriebsdaten und Kontextparameterdaten mit den entsprechenden Solldaten erfolgt und das Ergebnis des Vergleichs zumindest nachträglich zum Betrieb der Handdosiervorrichtung mit den sonstigen erfassten Werten und Daten über den Betrieb in Form von erweiterten Protokolldatensätzen erfasst und gespeichert werden kann.

Dadurch wird ermöglicht, dass unabhängig vom zeitlichen Abstand zwischen dem Betrieb der Handdosiervorrichtung und einer entsprechenden Abarbeitung einer Arbeitsroutine, eine Analyse stattfinden kann und eine Aussage getroffen werden kann, ob im Rahmen der Abarbeitung der Arbeitsroutine für genau eine Probe bzw. für genau eine Flüssigkeit alle erfassten Betriebsdaten und/oder Kontextparameterdaten die Sollvorgaben erfüllen oder im Bereich von Sollvorgaben liegen. Mit anderen Worten ausgedrückt bedeutet dies, dass die von der Dokumentationseinheit geschaffenen und gespeicherten Protokolldatensätze zur automatischen Validierung oder zur Selbstvalidierung der durchgeführten Arbeitsroutinen beitragen, da aus den Protokolldatensätzen selbst ersichtlich ist, ob und vor allem auch bzgl. welches Arbeitsschrittes oder bzgl. welches Kontextparameters oder dergleichen eine Abweichung zu den Sollvorgaben oder Soll-Daten vorliegt. Dies ermöglicht auch in besonders vorteilhafter Weise, dass eine Vergleichbarkeit von unterschiedlichen Proben oder Flüssigkeiten, die mit vermeintlich identischen Arbeitsroutinen erzeugt oder geschaffen werden, objektiviert oder überhaupt erst sinnvoll möglich wird, da eine Vergleichbarkeit erst angenommen werden kann, wenn die von der Dokumentationseinheit erzeugten und gespeicherten Protokolldatensätze keine oder keine signifikanten Abweichungen in den Vergleichsergebnissen der seitens der Vergleichseinheit angestellten Vergleiche aufweisen. Umgekehrt bedeutet dies, dass bei entsprechenden Abweichungen in den Vergleichen in zumindest einem Protokolldatensatz einer Probe oder Flüssigkeit die Vergleichbarkeit mit einer anderen Probe oder Flüssigkeit, die vermeintlich mittels einer identischen Arbeitsroutine erzeugt wurde, von vorne rein ausfällt oder ausgeschlossen werden kann. Dadurch wird in bisher unbekannter Weise die Arbeit in Laboren oder vergleichbaren Einrichtungen erleichtert, da insbesondere quantitative und qualitative Vergleichbarkeit oder eben der Ausschluss einer entsprechenden Vergleichbarkeit zwischen unterschiedlichen Proben oder Flüssigkeiten, die mittels vermeintlich identischer Arbeitsroutinen gewonnen oder erzeugt werden, ermöglicht wird.

Bei der Handdosiervorrichtung des vorliegenden Systems kann es sich um eine nach dem Luftpolsterprinzip arbeitende Dosiervorrichtung handeln, welche eine integrierte Kolben-Zylindereinheit aufweist. Alternativ kann die Handdosiervorrichtung jedoch auch als Mikrodosiervorrichtung ausgebildet sein, die über eine Mikromembranpumpe und/oder über einen Freistrahldosierer verfügt. Ebenfalls alternativ kann die Handdosiervorrichtung als Dispenser ausgebildet sein, der nach dem Direktverdrängerprinzip arbeitet und dessen Zylinder und Kolben als Verbrauchsgegenstände, insbesondere als Einmal-Vorrichtungen ausgestaltet sein können. Auch kann die Handdosiervorrichtung als Mehrkanal-Dosiervorrichtung ausgebildet sein. Die Netzwerkzugangseinrichtung des erfindungsgemäßen Systems kann beispielsweise als sog. Gateway ausgebildet sein, welches bidirektional mit der Handdosiervorrichtung datentechnisch kommuniziert. Für die datentechnische Verbindung können standardisierte Verbindungstechniken oder Verbindungsprotokolle, wie beispielsweise Bluetooth oder WLAN zum Einsatz kommen. Es kann jedoch auch vorgesehen sein, dass die datentechnische Verbindung zwischen der Netzwerkzugangseinrichtung und der Handdosiervorrichtung kabelgebunden bzw. über physische Kontaktmittel ausgebildet oder hergestellt wird. Dabei kann vorgesehen sein, dass die Netzwerkzugangseinrichtung und die Handdosiervorrichtung entsprechend komplementär zueinander ausgebildete Kontaktmittel, beispielsweise Stecker oder dergleichen aufweisen. Die Netzwerkzugangseinrichtung kann beispielsweise derart ausgestaltet sein, dass sie zum Empfang sowie zur Ausgabe von Daten in Form eines Netzwerkprotokolls eingerichtet ist und entsprechende Ausgänge und Eingänge für Daten aufweist. Beispielsweise kann vorgesehen sein, dass die Netzwerkzugangseinrichtung über eine Local-Area-Network-Verbindung (LAN-Verbindung) mit der Datenverarbeitungsanlage verbunden ist.

Die Datenverarbeitungsanlage selbst kann als Server-Client-System ausgebildet sein. Als Client kommen beispielsweise PCs, Tablets, Smartphones oder andere tragbare elektronische Geräte zum Einsatz. Alternativ kann vorgesehen sein, dass der oder die Clients als Augmented-Reality-Vorrichtungen ausgebildet sind und dazu eingerichtet sind Bildinhalte, insbesondere virtuelle Bildinhalte, zu erzeugen und mit realen Bildinhalten zu überlagern und eine Darstellung der überlagerten virtuellen und realen Bildinhalte zu erzeugen und auszugeben. Dabei kann vorgesehen sein, dass der oder die Clients die wesentliche datentechnische Infrastruktur, insbesondere Software umfassen. Alternativ kann auch vorgesehen sein, dass die wesentliche datentechnische Infrastruktur, insbesondere die Software, von dem Server umfasst ist und der oder die Clients lediglich zum Zugriff auf die serverseitige Infrastruktur eingerichtet sind.

Dementsprechend kann auch vorgesehen sein, dass die Vergleichseinheit und/oder die Dokumentationseinheit einem Server einer Datenverarbeitungsanlage oder einem oder mehreren Clients der Datenverarbeitungsanlage zugeordnet sind, also von diesen umfasst sind. Über die datentechnische Verbindung zwischen der Datenverarbeitungsanlage einerseits und der Handdosiervorrichtung über die zwischengeschaltete Netzwerkzugangseinrichtung, und die Kontextparametersensoreinheit andererseits, wird ermöglicht, dass die Betriebsdaten der Betriebssensoreinheit, die Kontextparameterdaten der Kontextparametersensoreinheit sowie die entsprechenden Sollbetriebsdaten und Sollkontextparameterdaten zwischen den einzelnen Einrichtungen des Systems weitestgehend frei übertragbar sind. Dadurch kann beispielsweise auch realisiert werden, dass sowohl die Datenverarbeitungsanlage als auch die Handdosiervorrichtung selbst über eine Vergleichseinheit verfügt. Andererseits kann dementsprechend auch realisiert werden, dass die Vergleichsergebnisse einer Vergleicheinheit, unabhängig von der Anordnung oder Zuordnung der Vergleichseinheit den sonstigen Systemkomponenten oder Einrichtungen des Systems, insbesondere der Dokumentationseinheit, aber auch anderen Systemkomponenten zur Verfügung gestellt werden.

Die Betriebssensoreinheit kann einen oder eine Vielzahl von Sensoren aufweisen, die den unmittelbaren Betrieb der Handdosiervorrichtung erfassen. Beispielsweise kann ein Sensor vorgesehen sein, der den Betrieb der Kolben-Zylinder-Einheit der Handdosiervorrichtung erfasst. Zusätzlich oder alternativ kann ein Sensor vorgesehen sein, der den Betrieb einer elektrischen Antriebsvorrichtung für die Kolben-Zylinder-Einheit erfasst. Weiter kann ein Bewegungssensor oder Trägheitssensor vorgesehen sein, der die Translation und/oder Rotation und/oder Beschleunigung der Handdosiervorrichtung erfasst. Die Betriebssensoreinheit kann aber auch dazu eingerichtet sein, statische Betriebsdaten zu erfassen. Beispielsweise kann vorgesehen sein, dass die Betriebssensoreinheit eine Seriennummer oder ein sonstiges Identifikationsmittel der Handdosiervorrichtung erfasst oder erkennt. Weitere Sensoren der Betriebssensoreinheit können beispielsweise als Betriebsstundenzähler, insbesondere als Betriebsstundenzähler in Abhängigkeit eines Kalibrierdatums der Handdosiervorrichtung oder als Kamera zur optischen Erfassung des Betriebs der Handdosiervorrichtung innerhalb oder außerhalb der Handdosiervorrichtung oder dergleichen ausgebildet sein.

Gemäß einer ersten vorteilhaften Ausgestaltung des Dosiersystems kann vorgesehen sein, dass dieses zumindest eine, insbesondere von der Handdosiervorrichtung umfasste Ausgabeeinheit zur Ausgabe von Benutzeranweisungen und/oder Benutzerhinweisen umfasst, wobei die Ausgabeeinheit derart mit der Vergleichseinheit verbunden ist, dass Vergleichsergebnisse, insbesondere eine im Rahmen des Vergleichs identifizierte Abweichung der Daten von den Solldaten, unmittelbar ausgegeben wird. Derartige Ausgabeeinheiten können für eine Vielzahl von Zwecken genutzt werden. Beispielsweise können über die Ausgabeeinheit auch andere Benutzeranweisungen und Benutzerhinweise ausgegeben werden, die beispielsweise eine Führung des Bedieners oder Benutzers im Rahmen der Abarbeitung einer Arbeitsroutine ermöglichen oder verbessern sollen. Dies bedeutet, dass die Ausgabeeinheit dazu eingerichtet sein kann, Benutzerhinweise auszugeben, die die Einhaltung der Sollbetriebsdaten und/oder Sollkontextparameterdaten erleichtern oder ermöglichen können. Die vorliegende Ausführungsvariante sieht für die Ausgabeeinheit aber auch vor, dass diese mit der Vergleichseinheit verbunden ist, sodass Vergleichsergebnisse, insbesondere die Abweichung der Daten von den Solldaten, an einen Bediener oder Benutzer ausgegeben werden können. Dies setzt jedoch voraus, dass das System, insbesondere beim Vorsehen lediglich einer einzelnen, zentralen Vergleichseinheit, möglichst alle Betriebs- und/oder Kontextparameterdaten sowie die dazugehörigen Solldaten unmittelbar erhält oder an die Vergleichseinheit übertragen werden und eine unmittelbare Auswertung also ein unmittelbarer Vergleich durchgeführt wird. Dies könnte jedoch auch eine ständige Datenübertragung zwischen der Handdosiervorrichtung und den restlichen Einrichtungen des Systems voraussetzen. Um die datentechnische Verbindung zwischen der Handdosiervorrichtung und den sonstigen Systemkomponenten effizient, insbesondere energieeffizient bzgl. des Energieverbrauchs der Handdosiervorrichtung, auszugestalten, kann dementsprechend alternativ vorgesehen sein, dass eine weitere Vergleichseinheit vorgesehen ist, welche der Handdosiervorrichtung zugeordnet und dementsprechend eine energieeffiziente interne datentechnische Kommunikation zwischen beispielsweise der Betriebssensoreinheit und einem Speicher mit Soll-Betriebsdaten ermöglicht, sodass zumindest für die Betriebsdaten bereits im Rahmen des Betriebs der Handdosiervorrichtung ein Vergleichsergebnis erstellt und basierend darauf Ausgaben mittels der Ausgabeeinheit generiert werden können, ohne dass eine externe Kommunikation der Handdosiervorrichtung über die Netzwerkzugangseinrichtung und den damit verbundenen sonstigen Systemkomponenten notwendig wird. Vorteilhaft kann vorgesehen sein, dass die seitens der Handdosiervorrichtung erzeugten Vergleichsergebnisse einer Vergleichseinheit interwallmäßig oder nach Beendigung des Betriebs der Handdosiervorrichtung, insbesondere nach Beendigung einer Arbeitsroutine an die Netzwerkzugangseinrichtung übermittelt und dementsprechend auch einer Dokumentationseinheit zugänglich gemacht werden. Dies bedeutet mit anderen Worten ausgedrückt, dass beispielsweise auch eine räumliche Aufteilung der Vergleichseinheit über die einzelnen Systemkomponenten sinnvoll sein kann, solange gewährleistet wird, dass zumindest nach Abschluss des Betriebs der Handdosiervorrichtung oder nach Abschluss der Durchführung einer Arbeitsroutine mittels der Handdosiervorrichtung die jeweiligen von den Vergleichseinheiten erstellten und gespeicherten Vergleichsergebnisse zusammengeführt und die jeweiligen Daten zu Protokolldatensätzen verknüpft werden.

Eine weitere besonders vorteilhafte Ausgestaltung des Dosiersystems sieht vor, dass die zumindest eine Kontaktparametersensoreinheit zur Erfassung statischer und/oder dynamischer Kontextparameter eingerichtet ist. Beispielsweise kann vorgesehen sein, dass die Kontextparametersensoreinheit zur Erfassung dynamischer Kontextparameter, beispielsweise einer Raumtemperatur in dem Labor oder Raum, in dem die Handdosiervorrichtung zum Einsatz kommt, eingerichtet ist. Alternativ kann die Kontextparametersensoreinheit auch statische Kontextparameter erfassen. Beispielsweise kann die Kontextparametersensoreinheit zur Erfassung einer Benutzeridentität eingerichtet sein und damit, die, zumindest für die Durchführung einer Arbeitsroutine, statische Identität eines Benutzers der Handdosiervorrichtung erfassen. Beispielsweise kann die Kontextparametersensoreinheit dazu eine Eingabeschnittstelle aufweisen, an der sich ein Benutzer über entsprechende Eingaben, beispielsweise über ein Alias und ein Kennwort identifiziert. Besonders vorteilhaft kann in diesem Zusammenhang vorgesehen sein, dass das System Mittel umfasst, die ermöglichen, dass ein nach einer Identifizierung eines Benutzers im System oder an einer Handdosiervorrichtung benutzerspezifische Systemeinstellungen geladen oder aufgerufen werden. Besonders bevorzugt kann vorgesehen sein, dass Mittel vorgesehen sind, mit denen benutzerspezifische Einstellungen oder Parameter für eine Handdosiervorrichtung aufgerufen und/oder an die Handdosiervorrichtung übertragen werden. Darüber hinaus können eine Vielzahl anderer statischer und/oder dynamischer Kontextparameter von der zumindest einen Kontextparametersensoreinheit erfasst werden. Beispielhaft seien an dieser Stelle den Luftdruck sowie weitere Wetterdaten genannt.

Gemäß einer weiteren, besonders bevorzugten Ausgestaltung des Systems kann vorgesehen sein, dass das System zumindest eine Klassifizierungseinheit umfasst, welche mit der Kontextparametersensoreinheit verbunden ist oder zumindest verbindbar ist und welche darüber hinaus dazu eingerichtet ist, dem erfassten Betrieb der Handdosiervorrichtung und/oder die erfassten Betriebsumweltbedingungen zu klassifizieren, wobei anhand der Klassifizierung ein Kontextparameter ableitbar und/oder Kontextparameterdaten erzeugbar sind. Die Klassifizierungseinheit kann somit einerseits dazu verwendet, dynamische Kontextparameter oder Kontextparameterdaten in statische oder quasistatische Kontextparameter und Kontextparameterdaten zu übersetzen. Anderseits kann die Klassifizierungseinheit auch dazu dienen, eine ansonsten einem Vergleich mit Solldaten nicht zugänglichen Betriebsumweltbedingung in einen klassifizierten Kontextparameter zu überführen und damit dem Vergleich einer Vergleichseinheit zugänglich zu machen. Beispielsweise kann vorgesehen sein, dass in der Klassifizierungseinheit für eine Anzahl registrierter Benutzer jeweils Benutzerklassen zugeordnet und gespeichert sind, sodass im Rahmen der Erfassung eines identifizierten Benutzers mittels der Kontextparametersensoreinheit eine Zuordnung des Benutzers zu einer bestimmten Benutzerklasse erfolgen kann und damit die jeweilige Benutzerklasse des aktuell an einer Handdosiervorrichtung registrierten Benutzers mit einer Soll-Benutzerklasse von der Vergleichseinheit verglichen werden kann. Dadurch kann erreicht werden, dass weitere Informationen und Daten bzgl. der Herstellung der Flüssigkeiten oder Behandlung der Proben, die einem Vergleich sonst nicht zugänglich sind, insbesondere nicht numerische Kontextparameter dem Vergleich einer Vergleichseinheit zugänglich gemacht und dementsprechend der Wert der resultierenden Protokolldatensätze weiter gesteigert wird. Grundsätzlich kann ebenfalls eine Klassifizierungseinheit auch mit der Betriebssensoreinheit verbunden sein und zur Klassifizierung von Betriebsdaten eingerichtet sein. Beispielsweise kann mit einer entsprechend ausgestalteten Klassifizierungseinheit eine Klassifizierung von Bewegungen oder Beschleunigungen der Handdosiervorrichtung, welche mittels eines entsprechenden Sensors erfasst werden, klassifiziert und einem Vergleich mittels der Vergleichseinheit zugänglich gemacht werden.

Erfindungsgemäß ist vorgesehen, dass das System eine Dokumentationsspeichereinrichtung umfasst, welche zur Speicherung und Katalogisierung von Protokolldatensätzen eingerichtet ist. Dadurch wird ermöglicht, dass die aussagekräftigen Protokolldatensätze, die erfindungsgemäß das Qualitäts-Management deutlich verbessern und vereinfachen und eine qualitative und quantitative Vergleichbarkeit von Proben und Flüssigkeiten sicherstellt, auch nachträglich, insbesondere über einen längeren Zeitraum hinweg. Darüber hinaus liefert auch die Katalogisierung der Protokolldatensätze mittels der Dokumentationsspeichereinrichtung besondere Vorteile für das System. Denn dadurch wird beispielsweise ermöglicht, dass die Protokolldatensätze einer Suchanfrage, insbesondere durch die Datenverarbeitungsanlage zugänglich gemacht werden. Weiter können die Protokolldatensätze durch die Katalogisierung mittels der Dokumentationsspeichereinrichtung auch nach ihrem Inhalt durchsucht werden.

Eine weitere, besonders vorteilhafte Ausgestaltung des Systems sieht eine Umwandlungseinrichtung vor, welche zur Umwandlung von Protokolldatensätzen in ein Datenformat eines elektronischen Laborbuchs oder zur Einbettung in Labor-Informations-Management-Systeme eingerichtet ist. Dadurch kann sichergestellt werden, dass die Protokolldatensätze einem möglichst weitreichenden Dokumentations- und/oder Auditierungssystem zur Verfügung gestellt werden, wobei besonders vorteilhaft gerade die von den Protokolldatensätzen umfassten Vergleichsergebnisse auch für die übergeordneten Systeme, insbesondere für ein Labor-Informations-Management-System von besonders hohem Wert sind, da diese eine sehr weitreichende und eindeutige Aussage zur Qualität der jeweiligen Proben oder Flüssigkeiten zulassen. Dementsprechend kann auch ein stetig steigender Bedarf nach Auditierbarkeit bekannter Dosiersysteme durch das Vorsehen der Umwandlungseinrichtung erreicht werden.

Ebenfalls kann besonders vorteilhaft eine Auswerteeinheit von dem Dosiersystem umfasst sein, wobei die Auswerteeinheit in besonders vorteilhafter Ausgestaltung mit der Dokumentationsspeichereinrichtung verbunden ist und zur Auswertung von Protokolldatensätzen und/oder zum Vergleich von Protokolldatensätzen eingerichtet ist und über eine Ausgabeschnittstelle Ergebnisse der Auswertung, insbesondere zur Weiterverarbeitung seitens des Dosiersystems, ausgeben kann. Dadurch wird auf vorteilhafte Art und Weise erreicht, dass die Protokolldatensätze einer nachträglichen Auswertung und einem nachträglichen Vergleich zugänglich sind und eine entsprechende Ausgabe an einen Bediener oder Benutzer erfolgen kann. Dies bedeutet beispielsweise, dass ein Benutzer im Vorfeld von weiteren Analyseschritten von hergestellten Proben oder Flüssigkeiten oder im Anschluss an weitere Analysetätigkeiten auch die Auswerteeinheit benutzten und anhand der entsprechenden Ausgabe einer Ausgabeschnittstelle eine Auswertung zur Qualität der jeweiligen Probe- oder Flüssigkeit vornehmen kann oder die Qualitäten unterschiedlicher Proben miteinander vergleichen kann. Dies dient beispielsweise dazu, festzustellen, ob unterschiedliche Proben oder Flüssigkeiten, aufgrund ihrer bisherigen Behandlung, insbesondere im Rahmen des erfindungsgemäßen Dosiersystems, überhaupt miteinander vergleichbar sind oder als gemeinsame Grundlage für qualitative oder quantitative Auswertungszwecke herangezogen werden können. Die Auswertungseinheit kann dabei sowohl stark automatisiert ausgebildet sein, sodass die Auswertung und/oder der Vergleich von Protokolldatensätzen weitestgehend ohne Zutun eines Bedieners oder Benutzers abläuft. Andererseits kann jedoch auch vorgesehen sein, dass die Auswerteeinheit von einem Benutzer oder Bediener beeinflusst oder gesteuert wird. Dies kann in besonders vorteilhafter Weise ermöglichen, dass die Auswertung und/oder der Vergleich auf spezielle Teile der Protokolldatensätze oder auf bestimmte Daten der Protokolldatensätze beschränkt oder gewichtet wird.

Gemäß einer weiteren besonders bevorzugten Ausgestaltung des Dosiersystems kann zudem vorgesehen sein, dass eine Programmiereinheit vorgesehen ist, welche bevorzugt von der Datenverarbeitungsanlage umfasst ist und welche zur Definition von Arbeitsroutinen und/oder zur Definition von Geräteparametern einer Parametereinheit der Handdosiervorrichtung eingerichtet ist, wobei die Programmiereinheit weiter dazu eingerichtet ist, auf der Grundlage von definierten Arbeitsroutinen und/oder Geräteparametern Sollbetriebsdaten und/oder Soll-Kontextparameterdaten zu erzeugen und zu speichern. Grundsätzlich kann die Programmiereinheit in vorteilhafter Weise realisieren, dass der Bediener oder Benutzer des Systems, insbesondere der Bediener oder der Benutzer der Handdosiervorrichtung für seine Zwecke maßgeschneiderte Arbeitsroutinen erstellen und Daten bzgl. der Arbeitsroutinen ggf. an die Handdosiervorrichtung übermitteln kann, sodass, insbesondere wenn die Programmiereinheit von der externen Datenverarbeitungsanlage umfasst ist, einerseits eine einfache Programmierung der Handdosiervorrichtung ermöglicht wird und andererseits ein besonders flexibler Einsatz der Handdosiervorrichtung ermöglicht wird. Neben Arbeitsroutinen kann die Programmiereinheit jedoch auch zur Definition von Geräteparametern einer Parametereinheit der Handdosiervorrichtung eingerichtet sein. Dies bedeutet, dass beispielsweise Kalibrierdaten für die Handdosiervorrichtung oder andere Geräteparameter, wie beispielsweise Steuerparameter für eine elektrische Antriebseinheit oder andere Daten durch die Programmiereinheit definiert und ggf. bei Bedarf an die Handdosiervorrichtung übermittelt oder übertragen und dort ggf. abermals gespeichert werden. Dadurch kann im weitesten Sinne eine Fernsteuerung oder genauer ausgedrückt eine Ferneinstellung der Handdosiervorrichtung erreicht werden.

Gemäß der bevorzugten Ausführungsform kann darüber hinaus jedoch auch vorgesehen sein, dass die Programmiereinheit dazu eingerichtet ist auf der Grundlage von definierten Arbeitsroutinen und/oder Geräteparametern Soll-Betriebsdaten und/oder Soll-Kontextparameter zu erzeugen und zu speichern. Dies bedeutet, dass wenn im Rahmen einer Definition einer Arbeitsroutine beispielsweise festgelegt wird, dass ein bestimmter Arbeitsschritt in einer bestimmten Zeit zu erfolgen hat oder dass eine bestimmte Arbeitsroutine nur in einem bestimmten Bereich einer Umgebungstemperatur abzulaufen hat die Programmiereinheit auf der Grundlage dieser oder ähnlicher Vorgaben dazu eingerichtet ist Soll-Daten zu erzeugen und zu speichern, die dann später während oder insbesondere nach der Durchführung der Arbeitsroutine mit den erfassten Betriebsdaten und/oder Kontextparameterdaten von der Vergleichseinheit verglichen werden können und über die Dokumentationseinheit den Protokolldatensätzen hinzugefügt werden können. Gleiches kann auch bei der Definition von Geräteparametern erfolgen. Beispielsweise kann die Programmiereinheit dazu eingerichtet sein den Betrieb der Handdosiervorrichtung mit Flüssigkeiten unterschiedlicher Eigenschaften, insbesondere unterschiedlicher Dichten und/oder unterschiedlicher Viskositäten zu ermöglichen, indem seitens der Programmiereinheit unterschiedliche Sätze von Geräteparametern erzeugt und gespeichert und für die Benutzung einer Handdosiervorrichtung mit einer entsprechenden Flüssigkeit an die Handdosiervorrichtung übertragen werden. Im Rahmen einer solchen Definition von Geräteparametern, beispielsweise für die Definition von Flüssigkeiten oder Flüssigkeitsklassen, kann ebenfalls vorgesehen sein, dass automatisch, zumindest aber halbautomatisch, beispielsweise durch eine Bestätigung im Rahmen einer Benutzerinteraktion, Soll-Daten, insbesondere Soll-Betriebsdaten erzeugt werden. Auch diese können während oder nach einem Betrieb des Systems, insbesondere einem Betrieb der Handdosiervorrichtung der Vergleichseinheit zusammen mit Betriebsdaten zugeführt und die Vergleichsergebnisse mittels der Dokumentationseinheit den Protokolldatensätzen zugeordnet werden.

Die Programmiereinheit kann darüber hinaus grundsätzlich dazu geeignet sein eine Liste von verfügbaren Arbeitsroutinen und/oder Geräteparametern oder Geräteparametersätzen über eine mit der Programmiereinheit verbundene Ausgabeschnittstelle auszugeben. Wie bereits erwähnt, kann die Programmiereinheit auch die Übertragung von entsprechenden Daten betreffend Arbeitsroutinen und/oder Geräteparametern an eine oder mehrere Handdosiervorrichtungen unterstützen oder veranlassen. Darüber hinaus kann die Programmiereinheit auch ein Austausch von definierten Arbeitsroutinen und/oder Geräteparametern oder Geräteparametersätzen mit anderen Dosiersystemen oder anderen Datenverarbeitungsanlagen ermöglichen.

Eine weitere besonders bevorzugte Ausgestaltung des Dosiersystems sieht vor, dass die Netzwerkzugangseinrichtung von einer Befestigungsvorrichtung zur lösbaren Befestigung von zumindest einer Handdosiervorrichtung umfasst ist. Die Befestigungsvorrichtung kann beispielsweise als Ständer ausgestaltet sein, der zur lösbaren Befestigung der Handdosiervorrichtung entsprechende Aufnahme- und/oder Befestigungsmittel aufweist. Bevorzugt kann vorgesehen sein, dass der Ständer zur Aufnahme und/oder Befestigung von mehr als einer Handdosiervorrichtung eingerichtet ist. Beispielsweise kann zudem vorgesehen sein, dass die Befestigungsvorrichtung über entsprechende Kontaktmittel verfügt, um eine kabelgebundene, datentechnische Verbindung zu den jeweils in den Aufnahme- und/oder Befestigungsmitteln angeordneten Handdosiervorrichtungen herzustellen.

Alternativ kann jedoch auch vorteilhaft vorgesehen sein, dass die Netzwerkzugangseinrichtung als separate Systemkomponente ausgebildet ist und alleinstehend oder frei-installierbar ist. Die kann insbesondere dann vorteilhaft sein, wenn für die Kommunikation, also für die datentechnische Verbindung zu der Handdosiervorrichtung ein standardisiertes Protokoll, wie beispielsweise WLAN, verwendet wird. Denn dann kann beispielsweise eine die Netzwerkzugangsvorrichtung als Systemkomponente des erfindungsgemäßen Systems gleichzeitig auch noch für andere Aufgaben und Tätigkeiten eingesetzt werden. Dies bedeutet umgekehrt auch, dass ggf bereits vorhandene Netzwerkzugangseinrichtungen in das erfindungsgemäße System eingebunden werden können. Damit können weitere Funktionalitäten des Systems, insbesondere über eine weiter reichende und verbesserte Konvektivität, erreicht und gleichzeitig die Systemeinrichtungs- und Unterhaltskosten reduziert werden. Dementsprechend kann vorteilhaft vorgesehen sein, dass die Netzwerkzugangseinrichtung als WLAN-Hub oder WLAN-Router ausgebildet ist.

Durch die Kombination oder Integration der Netzwerkzugangseinrichtung zu oder in die Befestigungsvorrichtung können verschiedene Vorteile erreicht werden. Einerseits kommen die besagten Befestigungsvorrichtungen in der Regel in der Nähe des Einsatz- und/oder Betriebsortes der Handdosiervorrichtung zum Einsatz, sodass für die, insbesondere kabellose datentechnische Verbindung zwischen der Netzwerkzugangseinrichtung und zumindest einer Handdosiervorrichtung nur eine verhältnismäßig kurze Übertragungsstrecke überbrückt werden muss. Außerdem können die Befestigungsvorrichtungen Mittel zum Aufladen von elektrischen Energiespeichern einer in der Befestigungsvorrichtung aufgenommenen und befestigten Handdosiervorrichtung aufweisen, wozu Energieübertragungsmittel, beispielsweise zum kabelgebundenen Übertragen von Energie oder zum induktiven Übertragen von Energie vorgesehen werden müssen. In diesem Fall können die Energieübertragungsmittel ggf. auch genutzt werden, um die datentechnische Verbindung zu der Handdosiervorrichtung herzustellen oder bereitzustellen.

Eine weitere, besonders bevorzugte Ausgestaltung des Dosiersystems sieht vor, dass die Kontextparametersensoreinheit zur Duplikation oder zur Erfassung eines Messwerts oder eines Ausgangssignals einer labortechnischen Flüssigkeits-Messvorrichtung eingerichtet ist. Als Flüssigkeits-Messvorrichtung kann dabei beispielsweise eine pH-Messvorrichtung, eine Volumen-Messvorrichtung, eine Dichte-Messvorrichtung, eine Füllstands-Messvorrichtung oder eine Masse-Messvorrichtung, beispielsweise eine Waage zum Einsatz kommen. Über die Duplizierung oder die Erfassung eines Messwerts oder eines Ausgangssignals einer solchen Messvorrichtung kann in besonders vorteilhafter Weise der mittelbare Betrieb der Handdosiervorrichtung und damit die Benutzung des Dosiersystems genau und aussagekräftig dokumentiert werden. Dementsprechend kann bei einem Vorsehen oder Definieren entsprechender Soll-Kontextparameterdaten zu den erfassten oder duplizierten Messwerten oder Ausgangssignalen im Rahmen des Vergleichs mit der Vergleichseinheit und der anschließenden Zuordnung der Vergleichsergebnisse zu den Protokolldatensätzen durch die Dokumentationseinheit eine sehr weitreichende Prozesskontrolle und ein sehr umfangreiches Qualitäts-Management mit dem erfindungsgemäßen System erreicht werden. Denn mit dem Abgreifen oder Erfassen der Messwerte oder Ausgangssignale der Messvorrichtung oder Messvorrichtungen kann die Eigenschaft und die Entwicklung der Proben oder Flüssigkeiten, über die unmittelbare Bedienung der Handdosiervorrichtung hinaus, erfasst und ausgewertet werden, was bei der Kombination der entsprechenden Daten mit den erlangten oder erlangbaren Vergleichsergebnissen zu aussagekräftigen Protokolldatensätzen führt und eine erhebliche Aussagekraft über die korrekte und/oder vollständige Durchführung von Arbeitsroutinen und/oder Kalibriervorgängen erlaubt. Außerdem kann dadurch auch die Analyse hinsichtlich der Vergleichbarkeit oder der Gleichwertigkeit von unterschiedlichen Proben oder Flüssigkeiten bzw. zwar nach vermeintlich identischen Arbeitsroutinen hergestellten aber in unterschiedlichen Ansätzen oder zeitlich zueinander versetzt durchgeführten Arbeitsroutinen hergestellten Proben und Flüssigkeiten verbessert werden. Beispielsweise kann also die Kontextparametersensoreinheit dazu eingerichtet sein einen Messwert einer pH-Wert-Messvorrichtung, welche den pH-Wert einer Probe oder Flüssigkeit im Rahmen der Benutzung des Dosiersystems und der Handdosiervorrichtung erfasst, dupliziert oder erfasst werden und durch die Vergleichseinheit während oder nach dem Betrieb der Handdosiervorrichtung mit dazugehörigen Soll-Kontextparameterdaten verglichen werden, wobei die Vergleichsergebnisse von der Dokumentationseinheit den sonstigen Daten und Informationen der Protokolldatensätze zugeordnet und mit den Protokolldatensätzen gespeichert werden.

Besonders bevorzugt kann vorgesehen sein, dass das System eine automatisierte Handhabungsvorrichtung zur Bewegung und Bedienung der Handdosiervorrichtung umfasst. Die Handhabungsvorrichtung kann dabei elektrische, pneumatische und/oder hydraulische Stell- und/oder Antriebseinheiten aufweisen. Bevorzugt ist die Handhabungsvorrichtung als Robotikvorrichtung, insbesondere als Roboterarm, ausgebildet.

Die Erfindung führt auch auf ein Verfahren zum Betrieb eines elektronischen Labor-Dosiersystems gemäß Anspruch 10 .

Auch durch das erfindungsgemäße Verfahren wird in besonders vorteilhafter Weise erreicht, dass nachträglich zu einem Betrieb einer entsprechenden Handdosiervorrichtung oder nachträglich zu dem jeweiligen Betrieb des Dosiersystems eine möglichst weitreichende Nachvollziehbarkeit über die ausgeführten Tätigkeiten, insbesondere über die Durchführung von Arbeitsroutinen möglicht wird, um schlussendlich für eine bestimmte Probe oder eine bestimmte Flüssigkeit Rückschlüsse auf deren ordnungsgemäße Bearbeitung oder Behandlung und damit Rückschlüsse auf deren Vergleichbarkeit mit anderen Proben und Flüssigkeiten zu ermöglichen. Dadurch wird in erfinderischer Weiterentwicklung gewährleistet, dass das Verfahren einen besonders hohen Grad an Qualitäts-Management für das Dosiersystem ermöglicht.

Durch das erfindungsgemäße Verfahren werden die oben bereits genannten vorteilhaften Wirkungen des erfindungsgemäßen Systems realisiert, auf welche an dieser Stelle verwiesen wird. Auch für die nachfolgend erläuterten Ausgestaltungen und vorteilhaften Weiterbildungen des Verfahrens wird, soweit entsprechende Ausführungsform des Systems vorangegangen bereits beschrieben wurden, zumindest bezüglich der vorteilhaften Wirkungen auf die vorangegangene Beschreibung verwiesen.

Gemäß einer ersten solchen vorteilhaften Verfahrensvariante kann vorgesehen sein, dass die Ausgabe von Benutzeranweisungen und/oder Benutzerhinweisen mittels zumindest einer insbesondere von der Handdosiervorrichtung umfassten, Ausgabeeinheit erfolgt, wobei die Ausgabeeinheit derart mit der Vergleichseinheit verbunden ist, dass Vergleichsergebnisse, insbesondere eine im Rahmen des Vergleichs identifizierte Abweichung der Daten von den Soll-Daten, unmittelbar ausgegeben werden. Wie oben bereits beschrieben, kann es dazu vorteilhaft sein die Betriebsdaten sowie die Kontextparameterdaten bereits während des Betriebs der Handdosiervorrichtung an eine Vergleichseinheit zu übermitteln. Alternativ kann jedoch auch vorgesehen sein, dass die Vergleichseinheit aufgeteilt wird oder dass mehrere Vergleichseinheiten vorgesehen werden, von denen zumindest eine oder ein Teil in der Handdosiervorrichtung angeordnet ist, sodass zumindest für die Betriebsdaten ohne eine Datenkommunikation mit den restlichen Systemkomponenten bereits während des Betriebs der Handdosiervorrichtung ein Vergleich stattfinden kann. Bevorzugt kann dabei auch vorgesehen sein, dass die Vergleichsdaten, insbesondere die Vergleichsergebnisse zunächst lokal in der Handdosiervorrichtung gespeichert werden und nur intervallweise oder periodisch, insbesondere erst nach Abschluss des Betriebs der Handdosiervorrichtung über die Netzwerkzugangseinrichtung den sonstigen Systemkomponenten zur Verfügung gestellt werden.

Die entsprechende Ausgabe von Benutzerhinweisen oder Benutzeranweisungen hat den Vorteil, dass ein Abweichen von den Soll-Daten unmittelbar erkannt wird und dem Benutzer mitgeteilt wird, sodass der entsprechende Betrieb der Handdosiervorrichtung, insbesondere die Durchführung einer Arbeitsroutine abgebrochen und ggf. neu gestartet werden kann. Selbst wenn dies nicht der Fall ist, kann über die erfindungsgemäß vorgesehene Dokumentationseinheit und der Verknüpfung von Betriebsdaten, Kontextparameterdaten und den zugehörigen Vergleichsergebnissen aus den Daten mit den Soll-Daten zumindest im Nachhinein, also nach Beendigung der Benutzung der Handdosiervorrichtung oder des Dosiersystems, ermittelt und ohne zeitliche Begrenzung festgestellt oder nachgehalten werden, ob die Arbeitsroutine korrekt oder im Rahmen gegebener Toleranzen durchgeführt wurde und ob dementsprechend die erzeugte oder hergestellte Probe oder Flüssigkeit einen Soll-Zustand erreicht hat und mit anderen einwandfrei hergestellten Proben und Flüssigkeiten qualitativ oder quantitativ vergleichbar ist.

Eine weitere, besonders bevorzugte Ausgestaltung des Verfahrens sieht die Erfassung statischer und/oder dynamischer Kontextparameter über zumindest eine Kontextparametersensoreinheit vor. Beispielsweise kann eine Umgebungstemperatur oder eine Benutzer- und/oder Bedieneridentität erfasst werden.

Ebenfalls kann das Verfahren bevorzugt vorsehen, dass eine Klassifikation des erfassten Betriebs der Handdosiervorrichtung und/oder der erfassten Betriebsumweltbedingungen mit zumindest einer mit der Kontextparametersensoreinheit verbundenen Klassifizierungseinheit erfolgt, wobei anhand der Klassifizierung ein Kontextparameter abgeleitet werden kann und/oder Kontextparameterdaten erzeugt werden können. Dies kann für das Verfahren, wie oben bereits angedeutet, immer dann von Vorteil sein, wenn ein erfasster Kontextparameter oder eine Betriebsumweltbedingung zunächst nicht oder aus sich heraus zunächst nicht für einen Vergleich mit einer Soll-Vorgabe geeignet ist. Beispielsweise kann die Klassifizierung zur Klassifizierung eines erfassten Benutzers und zum Vergleich mit einer vorgegebenen Benutzerklasse oder zum Vergleich mit einer Soll-Benutzerklasse herangezogen werden.

Erfindungsgemäß ist vorgesehen, dass eine Speicherung und Katalogisierung von Protokolldatensätzen mittels einer Dokumentationsspeichereinrichtung erfolgt. Dadurch kann erreicht werden, dass die Protokolldatensätze zu beliebigen späteren Zeitpunkten nach deren Erstellung aufgefunden, durchsucht, anhand von bestimmten Kriterien durchsucht oder aufgefunden und ggf. weiterverarbeitet oder aufbereitet werden können.

Gemäß einer weiteren besonders bevorzugten Verfahrensvariante kann vorgesehen sein, dass eine Umwandlung von Protokolldatensätzen in ein Datenformat eines elektronischen Laborbuchs oder zur Einbettung in Labor-Informations-Managementsysteme mittels einer Umwandlungseinrichtung erfolgt. Dadurch können die vorteilhaften Wirkungen der Protokolldatensätze, insbesondere durch die Dokumentationseinheit mit den erfassten Daten verknüpften Vergleichsergebnisse auch über das Dosiersystem hinaus, genutzt werden oder nutzbar gemacht werden.

Eine weitere Ausgestaltungsform des Verfahrens sieht die Auswertung von Protokolldatensätzen und/oder den Vergleich von Protokolldatensätzen mittels einer Auswerteeinheit vor, welche mit einer Dokumentationsspeichereinrichtung verbunden ist, wobei eine Ausgabe der Ergebnisse der Auswertung, insbesondere zur Weiterverarbeitung seitens des Dosiersystems, über eine Ausgabeschnittstelle des Dosiersystems erfolgt. Dadurch kann erreicht werden, dass einzelne Proben oder Flüssigkeiten im Nachgang zu deren Herstellung oder Verarbeitung dahingehend geprüft und/oder verglichen werden, ob die Soll-Bedingungen eingehalten wurden und ob dementsprechend von einer Aussagekraft der Analyse der Proben und Flüssigkeiten sowie von einer Vergleichbarkeit unterschiedlicher Proben oder Flüssigkeiten ausgegangen werden kann.

Gemäß einer weiteren Verfahrensvariante ist die Definition von Arbeitsroutinen und/oder die Definition von Geräteparametern einer Parametereinheit der Handdosiervorrichtung mit einer, insbesondere von der Datenverarbeitungsanlage umfassten, Programmiereinheit vorgesehen, wobei die Programmiereinheit auf der Grundlage von definierten Arbeitsroutinen oder Geräteparametern Soll-Betriebsdaten und/oder Soll-Kontextparameterdaten erzeugt und/oder speichert.

Dadurch wird einerseits ein möglichst vielseitiger Einsatz des Dosiersystems ermöglicht, da flexible Arbeitsroutinen vorprogrammiert und ggf. an die Handdosiervorrichtung übertragen werden können. Gleichzeitig wird jedoch die Durchführung des Vergleichs zwischen Kontextparameterdaten und Betriebsdaten mit entsprechenden Soll-Daten erleichtert, da die Programmierung der Arbeitsroutinen und/oder der Geräteparameter automatisch, zumindest aber halbautomatisch bereits zur Definition oder Festlegung von Soll-Betriebsdaten und/oder Soll-Kontextparameterdaten führt, die dann mit den erfassten Betriebsdaten und Kontextparameterdaten verglichen und das Vergleichsergebnis gespeichert werden kann. Die Programmiereinheit kann dabei so ausgeführt sein, dass Arbeitsroutinen neu erstellt und/oder geändert werden können. Außerdem kann die Programmiereinheit so ausgeführt sein, dass erstellte oder geänderte Arbeitsroutinen einer automatischen Prüfung und/oder Validierung unterzogen werden.

Vorteilhaft kann vorgesehen sein, dass die mit der Programmiereinheit erstellten oder geänderten Arbeitsroutinen und/oder Geräteparameter sowie Sätze von Geräteparameter in einer entsprechenden Speichereinrichtung gespeichert und verwaltet werden können. Zudem kann vorgesehen sein, dass Daten, welche zur Ausführung der Arbeitsroutine nötig sind, als auch die Geräteparameter, die beispielsweise für eine Benutzung der Handdosiervorrichtung mit unterschiedlichen Flüssigkeitsklassen benötigt werden, von der Programmiereinheit über die entsprechenden datentechnischen Verbindungen an die Handdosiervorrichtung, insbesondere einen entsprechenden Speicher der Handdosiervorrichtung übertragen und von einer Steuereinheit der Handdosiervorrichtung aufgerufen oder genutzt werden.

Eine weitere Ausgestaltungsvariante des Verfahrens sieht die Duplikation oder die Erfassung eines Messwerts oder eines Ausgangssignals einer labortechnischen Flüssigkeits-Messvorrichtung mit zumindest einer Kontextparametersensoreinheit vor. Dadurch kann die labortechnische Umgebung des Dosiersystems optimal mit in das durch das Dosiersystem geschaffene Qualitäts-Management miteingebunden werden. Insbesondere können Kontextparameter, welche zumindest mittelbar auf den Betrieb des Dosiersystems zurückgehen oder mit dem Betrieb des Dosiersystems verbunden sind, ebenfalls im Rahmen eines entsprechenden Vergleiches und einer Speicherung der Vergleichsergebnisse zur langfristigen, weitreichenden und aussagekräftigen Dokumentation der durchgeführten Arbeitsroutinen verwendet werden und können besonders aufschlussreiche Informationen über die Validität oder die korrekte Herstellung der Proben und Flüssigkeit bereitstellen, wobei insbesondere eine automatische Verknüpfung zu den Protokolldatensätzen einen besonderen Mehrwert liefert, da die Protokolldatensätze insgesamt eindeutig einer Probe oder Flüssigkeit zuzuordnen sind oder zugeordnet werden können.

Vorteilhafte Ausgestaltungen und Varianten der Erfindung ergeben sich aus der nachfolgenden Beschreibung bevorzugter Ausführungsbeispiele sowie anhand der Zeichnungen.

Diese zeigen in:
- Fig. 1:: eine schematische Darstellung eines erfindungsgemäßen Dosiersystems;
- Fig. 2:: eine schematische Darstellung eines Ablaufs eines erfindungsgemäßen Verfahrens.

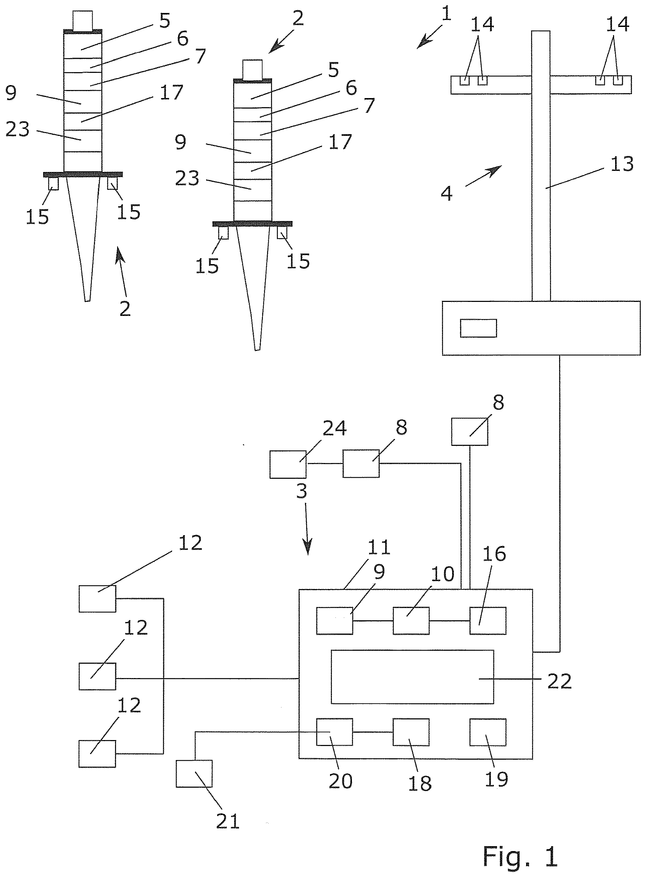

Die Fig. 1 zeigt ein Dosiersystem 1, welches eine Mehrzahl von Handdosiervorrichtungen 2, eine Datenverarbeitungsanlage 3 und eine Netzwerkzugangseinrichtung 4 aufweist. Die Handdosiervorrichtungen 2 umfassen jeweils eine Betriebssensoreinheit 5, die den Betrieb der Handdosiervorrichtungen 2 erfasst und diesbezügliche Betriebsdaten erzeugt und in einer Speichereinrichtung 6 der Handdosiervorrichtungen 2 speichert. Zudem umfassen die Handdosiervorrichtungen 2 jeweils ein Schnittstellenmodul 7, mit dem die Handdosiervorrichtungen 2, insbesondere zur Übertragung von Betriebsdaten über eine datentechnische Verbindung mit der Netzwerkzugangseinrichtung 4 verbindbar ist, wobei die Netzwerkzugangseinrichtung 4 mit der Datenverarbeitungsanlage 3 datentechnisch verbindbar ist, und wobei die Datenverarbeitungsanlage 3 ihrerseits datentechnisch mit zumindest einem den Betrieb der Handdosiervorrichtungen 2 zumindest mittelbar erfassenden und/oder Betriebsumweltbedingungen der Handdosiervorrichtungen 2 erfassenden und daraus Kontextparameterdaten erzeugenden Kontextparametersensoreinheit 8 verbindbar ist, wobei das Dosiersystem 1, insbesondere die Datenverarbeitungsanlage 3, eine Vergleichseinheit 9 aufweist, welche zumindest zum Vergleich von Betriebsdaten der Betriebssensoreinheit 5 mit Soll-Betriebsdaten und/oder Kontextparameterdaten der Kontextparametersensoreinheit 8 mit Soll-Kontextparametern und zur Erzeugung eines Vergleichsergebnisses, insbesondere einer im Rahmen des Vergleichs identifizierten Abweichung der Daten von den Soll-Daten, eingerichtet ist.

Weiter ist vorgesehen, dass das Dosiersystem 1, beispielsweise die Datenverarbeitungsanlage 3 des Dosiersystems 1, eine Dokumentationseinheit 10 umfasst, welche zur Erzeugung und Speicherung von Protokolldatensätzen betreffend den Betrieb der zumindest einen Handdosiervorrichtung 2, umfassend zumindest die Betriebsdaten und die Kontextparameterdaten, eingerichtet ist, wobei die Dokumentationseinheit 10 derart mit der Vergleichseinheit 9 verbunden ist, dass der Dokumentationseinheit 10 die Vergleichsergebnisse der Vergleichseinheit 9 übermittelbar sind und die Dokumentationseinheit 10 dazu eingerichtet ist die Vergleichsergebnisse als Teil der Protokolldatensätze den jeweiligen Betriebsdaten und Kontextparameterdaten zugeordnet, zu speichern, insbesondere nach Ende eines Betriebs der Handdosiervorrichtungen 2 zu ergänzen.

Im Beispiel der Fig. 1 ist die Datenverarbeitungsanlage 3 als Server-Clientsystem ausgebildet, welche einen Server 11 und eine Mehrzahl von Clients 12 umfasst, die ebenfalls miteinander datentechnisch verbunden sind. Der Server 11 umfasst im Beispiel der Darstellung der Fig. 1 die Vergleichseinheit 9 sowie die Dokumentationseinheit 10. Die Clients 12 können beispielsweise als mobile elektronische Geräte, beispielsweise als Tablets od.dgl. ausgeführt sein. Die Clients 12 können einen entsprechenden Zugriff auf den Server 11, insbesondere über eine oder mehrere grafische Benutzeroberflächen erlauben.

Die Netzwerkzugangseinrichtung 4 kann beispielsweise als Befestigungsvorrichtung 13 zur lösbaren Befestigung von zumindest einer Handdosiervorrichtung 2 ausgebildet sein. Die datentechnische Verbindung bzw. die Kommunikation zwischen den Handdosiervorrichtungen 2 und der Netzwerkzugangseinrichtung 4 kann beispielsweise über WLAN oder Bluetooth erfolgen. Alternativ kann auch vorgesehen sein, dass die Netzwerkzugangseinrichtung 4 über Kontaktmittel 14 verfügt, welche komplementär zu Kontaktmitteln 15 der Handdosiervorrichtungen 2 ausgebildet sind, sodass zusätzlich oder alternativ zu einer Drahtlosverbindung zwischen den Handdosiervorrichtungen 2 und der Netzwerkzugangseinrichtung 4 auch eine kabelgebundene Verbindung hergestellt werden kann. Die Verbindung kann einerseits zur Energieübertragung auf einen in der Fig. 1 nicht dargestellten Energiespeicher der Handdosiervorrichtungen 2 dienen. Zusätzlich kann die über die Kontaktmittel 14, 15 hergestellte Verbindung auch zur datentechnischen Verbindung zwischen der Netzwerkzugangseinrichtung 4 und den Handdosiervorrichtungen 2 dienen.

Die Datenverarbeitungsanlage 3, insbesondere der Server 11 kann in dem beispielhaften Dosiersystem 1 eine mit der Kontextparametersensoreinheit 8 verbundene Klassifizierungseinheit 16 aufweisen, welche dazu eingerichtet ist, den erfassten Betrieb der Handdosiervorrichtung 2 und/oder die erfassten Betriebsumweltbedingungen zu klassifizieren, wobei anhand der Klassifizierung ein Kontextparameter ableitbar und/oder Kontextparameterdaten erzeugbar sind. Beispielsweise kann vorgesehen sein, dass über die Kontextparametersensoreinheit 8 ein Benutzer identifiziert und über die Klassifizierungseinheit 16 einer Benutzerklasse zugeordnet wird.

Die Handdosiervorrichtungen 2 des Dosiersystems 1 können auch eine Ausgabeeinheit 17 zur Ausgabe von Benutzeranweisungen und/oder Benutzerhinweisen umfassen, wobei die Ausgabeeinheit 17 derart mit der Vergleichseinheit 9 verbunden ist, dass Vergleichsergebnisse, insbesondere eine im Rahmen des Vergleichs identifizierte Abweichung der Daten von Solldaten, unmittelbar ausgegeben wird. Wie oben bereits beschrieben, kann zur Vermeidung oder zur Verminderung von energieintensiver Kommunikation zwischen den Handdosiervorrichtungen 2 und der Netzwerkzugangseinrichtung 4 ebenfalls vorgesehen sein, dass die Handdosiervorrichtungen 2 ihrerseits über eine Vergleichseinheit 9 verfügen, die zumindest für die mit der Betriebssensoreinheit 5 erfassten Betriebsdaten einen Vergleich mit Soll-Betriebsdaten ausführt und ggf. eine Abweichung als Vergleichsergebnis über die Ausgabeeinheit 17 ausgibt.

Die Datenverarbeitungsanlage 3 verfügt zudem über eine Dokumentationsspeichereinrichtung 18, welche zur Speicherung und Katalogisierung von Protokolldatensätzen eingerichtet ist. Ebenfalls kann von der Datenverarbeitungsanlage 3, insbesondere von dem Server 11 eine Umwandlungseinrichtung 19 umfasst sein, welche zur Umwandlung von Protokolldatensätzen in ein Datenformat eines elektronischen Laborbuchs oder zur Einbettung in ein Labor-Informations-Management-System eingerichtet ist. Zudem kann das Dosiersystem 1 in der Ausgestaltung der Darstellung der Fig. 1 eine Auswerteeinheit 20 umfassen, welche mit der Dokumentationsspeichereinrichtung 18 verbunden ist und zur Auswertung von Protokolldatensätzen und/oder zum Vergleich von Protokolldatensätzen eingerichtet ist und über eine Ausgabeschnittstelle 21 Ergebnisse der Auswertung, insbesondere zur Weiterverarbeitung seitens des Dosiersystems, ausgeben kann. Die Datenverarbeitungsanlage 3, insbesondere der Server 11, kann zudem eine Programmiereinheit 22 umfassen, die zur Definition von Arbeitsroutinen und/oder zur Definition von Geräteparametern einer Parametereinheit 23 der Handdosiervorrichtung 2 eingerichtet ist, wobei die Programmiereinheit 22 dazu eingerichtet ist, auf der Grundlage von definierten Arbeitsroutinen und/oder Geräteparametern Soll-Betriebsdaten und/oder Soll-Kontextparameterdaten zu erzeugen und zu speichern.

Im Beispiel der Darstellung der Fig. 1 umfasst das Dosiersystem 1 eine zweite Kontextparametersensoreinheit 8, welche zur Duplikation oder zur Erfassung eines Messwerts oder eines Ausgangssignals einer labortechnischen Flüssigkeits-Messvorrichtung 24 eingerichtet ist.

Neben diesen Komponenten kann das Dosiersystem eine Vielzahl weiterer, vorteilhafter Komponenten aufweisen, von denen nachfolgend stellvertretend einige wenige skizziert werden sollen.

Das Dosiersystem 1, insbesondere die Handdosiervorrichtungen und die Datenverarbeitungsanlage können Mittel und Vorrichtungen zum sicheren Lokalisieren der Handdosiervorrichtung 2 aufweisen. Die Datenverarbeitungsanlage 3 kann zudem Mittel und Vorrichtungen aufweisen, die eine Reservierung oder eine Buchung der Handdosiervorrichtungen 2 erlauben. Bevorzugt können Benutzer über die Clients 12 und dem entsprechenden Zugriff auf den Server 11 von den Clients 12 aus Handdosiervorrichtungen 2 für eine Benutzung reservieren. Außerdem kann die Datenverarbeitungsanlage 3 die erfassten Betriebsdaten und/oder die Kontextparameterdaten mittels anderer Vorrichtungen des Dosiersystems weiter verarbeiten. Beispielsweise können die Betriebsdaten von der Datenverarbeitungsanlage 3 derart gespeichert und aufbereitet werden, dass eine Wartungs- und/oder Verwaltungseinheit einen aktuellen Zustand der Handdosiervorrichtungen 2 erfasst und ggf. Voraussagen über einen zukünftigen Zustand, insbesondere über zukünftige Verschleiß- und/oder Ermüdungserscheinungen der Handdosiervorrichtungen 2 trifft.

Das Dosiersystem 1 kann auch weitere, insbesondere von der Datenverarbeitungsanlage 3 umfasste Einheiten aufweisen, welche auf die oben bereits beschriebenen registrierten und klassifizierten oder kategorisierten Nutzer und die entsprechenden Daten zurückgreift. Beispielsweise kann eine nach Benutzerklasse abgestufte Funktionalität der Clients 12 vorgesehen sein, die sich wiederum in einer nutzerklassenabhängigen Bedienung oder nutzerklassenabhängigen Zugriffsrechten auf die Programmiereinheit 22, Auswerteeinheit 20 und/oder Dokumentationsspeichereinrichtung 18 niederschlägt.

Die Betriebsdatensensoreinheit 5 der Handdosiervorrichtungen 2 können beispielsweise Hubbewegungen einer Kolbeneinheit der Handdosiervorrichtung 2 erfassen oder den Zustand einer Antriebseinheit der Handdosiervorrichtung 2 erfassen. Alternativ oder zusätzlich kann die Betriebssensoreinheit 5 auch die Bewegung und Beschleunigungen der Handdosiervorrichtung 2 selbst erfassen. Die Kontextparametersensoreinheiten 8 können verschiedenste Umweltbedingungen erfassen, die im Rahmen des Einsatzes des Dosiersystems 1 von Relevanz sein können. Dazu gehören einerseits Flüssigkeitseigenschaften der verarbeiteten Flüssigkeit, Umgebungsparameter und ähnliches mehr.

Wie bereits angedeutet, kann über die Clients 12 ein Zugriff auf die Programmiereinheit 22 gewährleistet werden, um Arbeitsroutinen oder Geräteparameter zu erstellen und/oder zu ändern. Nach einer entsprechenden Erstellung oder Änderung der Arbeitsroutinen oder Geräteparameter können diese, ggf. abhängig von einer Berechtigungsklasse eines Benutzers, an eine oder mehrere Handdosiervorrichtungen 2 übertragen werden. Zusammen mit der Erzeugung oder Änderung von Arbeitsroutinen oder Geräteparameter können automatisch oder halbautomatisch von der Datenverarbeitungsanlage, insbesondere von dem Server 11, besonders bevorzugt von der Programmiereinheit 22 Solldaten erzeugt und abgespeichert werden. Diese können dann im Rahmen des Verfahrens mit den erfassten Betriebsdaten und Kontextparameterdaten verglichen und das Vergleichsergebnis zusammen mit den Betriebsdaten und Kontextparameterdaten in der Dokumentationseinheit 10 gespeichert werden. Die Solldaten können jedoch auch auf anderem Wege, insbesondere durch eine manuelle benutzerseitige Vorgabe, beispielsweise über die Clients 12 definiert und gespeichert werden.

Für eine drahtlose Kommunikation zwischen den Handdosiervorrichtungen 2 und der Netzwerkzugangseinrichtung 4 kann beispielsweise eine Übertragung nach einem Standard, wie beispielsweise Bluetooth oder WLAN erfolgen. Als Schnittstellenprotokoll kann beispielsweise JSON-RPC oder aber MQTT zum Einsatz kommen. Besonders bevorzugt ist dabei sichergestellt, dass die Schnittstellenmodule 7 der Handdosiervorrichtungen 2 eine sichere Anmeldung und Authentifizierung in einem Drahtlosnetzwerk der Dosiervorrichtung 1 ermöglichen.

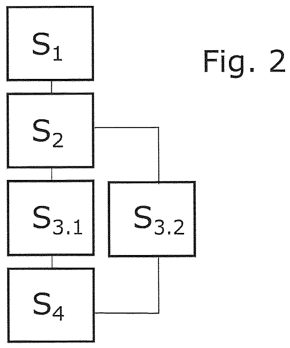

In der Fig. 2 wird ein beispielhaftes Verfahren zum Betrieb eines elektronischen Labor-Dosiersystems skizziert. Es kann beispielsweise vorgesehen sein, dass das Verfahren damit beginnt, dass ein Benutzer an der Handdosiervorrichtung 2 in einem ersten Verfahrensschritt S₁ eine Flüssigkeit oder eine Flüssigkeitsklasse auswählt und eine durchzuführende Arbeitsroutine bestimmt. Die Flüssigkeit oder Flüssigkeitsklasse kann durch Geräteparameter definiert werden, welche in der Parametereinheit 23 gespeichert sind, und die ggf. zuvor von der Programmiereinheit 22 an die Handdosiervorrichtung 2 übermittelt wurden. Auch die Daten bzgl. eines durchzuführenden Arbeitsablaufs oder einer durchzuführenden Arbeitsroutine können von einer Speichereinrichtung der Handdosiervorrichtung gespeichert sein und ebenfalls im Vorfeld zum Verfahrensschritt S₁ mit der Programmiereinheit 22 festgelegt und an eine Speichereinrichtung der Handdosiervorrichtung 2 übertragen worden sein. Die Auswahl der Flüssigkeit oder Flüssigkeitsklasse sowie die Auswahl der Arbeitsroutine im Schritt S₁ kann bereits einer Berechtigungs- oder Zugriffskontrolle unterliegen, in der unter Umständen unter Einbeziehung der Datenverarbeitungsanlage 3 überprüft wird, ob beispielsweise ein aktuell für oder an der Handdosiervorrichtung 2 registrierter oder angemeldeter Benutzer eine entsprechende Berechtigung oder Berechtigungsklasse zur Handhabung einer Flüssigkeit oder einer Flüssigkeitsklasse aufweist, ob der angemeldete oder registrierte Benutzer eine ausreichende Kompetenz- oder Schulungsklasse zur Durchführung des gewählten Arbeitsablaufs oder zur Durchführung der gewählten Arbeitsroutine aufweist, ob die Handdosiervorrichtung, mit der gearbeitet werden soll, eine der Arbeitsroutine entsprechende oder für die Durchführung der Arbeitsroutine vorausgesetzte Kalibrierung, Aktualität der Kalibrierung und/oder Geräteklasse aufweist.

In einem folgenden Verfahrensschritt S₂ beginnt der Benutzer mit der Durchführung der jeweiligen Tätigkeiten, insbesondere mit der Abarbeitung einer Arbeitsroutine unter Verwendung der Handdosiervorrichtung 2. Dabei können über eine Ausgabeeinheit 17 Anweisungen und Hinweise ausgegeben werden, die die Befolgung und/oder Einhaltung der Arbeitsroutine erleichtern. Darüber hinaus kann während des Schritts S₂ die Betriebssensoreinheit 5 sowie die Kontextparametersensoreinheiten 8 Betriebsdaten erfassen und speichern sowie Kontextparameterdaten erfassen und speichern. Im Beispiel der Fig. 2 soll das Verfahren so ausgestaltet sein, dass während des Verfahrensschritts S₂ die Betriebsdaten und die Kontextparameterdaten zunächst nur gesammelt oder erfasst werden. Nach Abschluss des Verfahrensschritts S₂ folgt ein weitere Verfahrensschritt S_{3.1}, in dem über die jeweiligen datentechnischen Verbindungen die erfassten Betriebsdaten und Kontextparameterdaten einer Dokumentationseinheit 10 zugeführt werden, und in Protokolldatensätzen oder in einem Protokolldatensatz gespeichert werden. In einem im Beispiel der Fig. 2 parallel zum Verfahrensschritt S_{3.1} ablaufenden Verfahrensschritt S_{3.2}, der aber durchaus dem Verfahrensschritt S_{3.1} auch vor- oder nachgeschaltet sein kann, werden die Betriebsdaten und die Kontextparameterdaten einer Vergleichseinheit 9 zugeführt oder zur Verfügung gestellt. Neben den Betriebsdaten und Kontextparameterdaten wird im Verfahrensschritt S_{3.2} die Vergleichseinheit 9 auch mit Soll-Betriebsdaten und Soll-Kontextparameterdaten versorgt.

Ebenfalls im Verfahrensschritt S_{3.2} erfolgt ein Vergleich zwischen den Daten und den Solldaten, wobei Vergleichsergebnisse erzeugt werden, die insbesondere eine Abweichung zwischen den Daten und den Solldaten wiedergeben. In einem anschließenden Verfahrensschritt S₄ werden die im Verfahrenschritt S_{3.2} erhaltenen Vergleichsergebnisse den im Verfahrensschritt S_{3.1} erzeugten Protokolldatensätzen zugeordnet.

### Bezugszeichenliste

- 1: Dosiersystem
- 2: Handdosiervorrichtung
- 3: Datenverarbeitungsanlage
- 4: Netzwerkzugangseinrichtung
- 5: Betriebssensoreinheit
- 6: Speichereinrichtung
- 7: Schnittstellenmodul
- 8: Kontextparametersensoreinheit
- 9: Vergleichseinheit
- 10: Dokumentationseinheit
- 11: Server
- 12: Client
- 13: Befestigungsvorrichtung
- 14: Kontaktmittel
- 15: Kontaktmittel
- 16: Klassifizierungseinheit
- 17: Ausgabeeinheit
- 18: Dokumentationsspeichereinrichtung
- 19: Umwandlungseinrichtung
- 20: Auswerteeinheit
- 21: Ausgabeschnittstelle
- 22: Programmiereinheit
- 23: Parametereinheit
- 24: Flüssigkeits-Messvorrichtung
- S₁: Verfahrensschritt
- S₂: Verfahrensschritt
- S_{3.1}, S_{3.2}: Verfahrensschritt
- S₄: Verfahrensschritt

## Patentansprüche

1. Elektronisches Labor-Dosiersystem für Flüssigkeiten umfassend
zumindest eine elektrische Handdosiervorrichtung (2) die zumindest eine Betriebssensoreinheit (5) aufweist, die den Betrieb der Handdosiervorrichtung (2) erfasst und diesbezügliche Betriebsdaten erzeugt und insbesondere in einer Speichereinrichtung (6) der Handdosiervorrichtung (2) speichert, wobei die Handdosiervorrichtung (2) ein Schnittstellenmodul (7) aufweist, mit dem die Handdosiervorrichtung (2), insbesondere zur Übertragung der Betriebsdaten, über eine datentechnische Verbindung mit einer externen Netzwerkzugangseinrichtung (4) verbindbar ist, wobei die Netzwerkzugangseinrichtung (4) mit einer externen Datenverarbeitungsanlage (3) datentechnisch verbindbar ist, wobei die Datenverarbeitungsanlage (3) datentechnisch mit zumindest einem den Betrieb der Handdosiervorrichtung (2) zumindest mittelbar erfassenden und/oder Betriebsumweltbedingungen der Handdosiervorrichtung (2) erfassenden und daraus Kontextparameterdaten erzeugenden Kontextparametersensoreinheit (8) verbindbar ist, wobei das Dosiersystem (1), insbesondere die Datenverarbeitungsanlage (3), eine Vergleichseinheit (9) aufweist, welche zumindest zum Vergleich von Betriebsdaten der Betriebssensoreinheit (5) mit Soll-Betriebsdaten und/oder Kontextparameterdaten der Kontextparametersensoreinheit (8) mit Soll-Kontextparameterdaten und zur Erzeugung eines Vergleichsergebnisses, insbesondere einer im Rahmen des Vergleichs identifizierten Abweichung der Daten von den Solldaten, eingerichtet ist,
**dadurch gekennzeichnet**
**dass** das Dosiersystem (1) eine Dokumentationseinheit (10) umfasst, welche zur Erzeugung und Speicherung von Protokolldatensätzen betreffend den Betrieb der zumindest einen Handdosiervorrichtung (2) umfassend zumindest die Betriebsdaten und die Kontextparameterdaten, eingerichtet ist, wobei die Dokumentationseinheit (10) derart mit der Vergleichseinheit (9) verbunden ist, dass der Dokumentationseinheit (10) die Vergleichsergebnisse der Vergleichseinheit (9) übermittelbar sind und die Dokumentationseinheit (10) dazu eingerichtet ist die Vergleichsergebnisse als Teil der Protokolldatensätze den jeweiligen Betriebsdaten und Kontextparameterdaten zugeordnet, zu speichern, insbesondere nach Ende eines Betriebs der Handdosiervorrichtung (2) zu ergänzen, ferner umfassend eine Dokumentationsspeichereinrichtung (18), welche zur Speicherung und Katalogisierung von Protokolldatensätzen eingerichtet ist.

2. Dosiersystem nach Anspruch 1,
**gekennzeichnet durch**
zumindest eine, insbesondere von der Handdosiervorrichtung (2) umfasste, Ausgabeeinheit (17) zur Ausgabe von Benutzeranweisungen und/oder Benutzerhinweisen, wobei die Ausgabeeinheit (17) derart mit der Vergleichseinheit (9) verbunden ist, dass Vergleichsergebnisse, insbesondere eine im Rahmen des Vergleichs identifizierte Abweichung der Daten von den Solldaten, unmittelbar ausgegeben wird.

3. Dosiersystem nach Anspruch 1 oder 2,
**dadurch gekennzeichnet,**
**dass** zumindest eine Kontextparametersensoreinheit (8) zur Erfassung statischer und/oder dynamischer Kontextparameter eingerichtet ist.

4. Dosiersystem nach einem der Ansprüche 1 bis 3 ,
**dadurch gekennzeichnet,**
**dass** zumindest eine Kontextparametersensoreinheit (8) mit einer Klassifizierungseinheit (16) verbunden ist, welche dazu eingerichtet den erfassten Betrieb der Handdosiervorrichtung (2) und/oder die erfassten Betriebsumweltbedingungen zu klassifizieren, wobei anhand der Klassifizierung ein Kontextparameter ableitbar und/oder Kontextparameterdaten erzeugbar sind.

5. Dosiersystem nach einem der Ansprüche 1 bis 4,
**gekennzeichnet durch**
eine Umwandlungseinrichtung (19), welche zur Umwandlung von Protokolldatensätzen in ein Datenformat eines elektronischen Laborbuchs oder zur Einbettung in Labor-Informations-Management-System eingerichtet ist.

6. Dosiersystem nach einem der Ansprüche 4 oder 5,
**gekennzeichnet durch**
zumindest eine Auswerteeinheit (20), welche mit der Dokumentationsspeichereinrichtung (18) verbunden ist und zur Auswertung von Protokolldatensätzen und/oder zum Vergleich von Protokolldatensätzen eingerichtet ist und über eine Ausgabeschnittstelle (21) Ergebnisse der Auswertung, insbesondere zur Weiterverarbeitung seitens des Dosiersystems, ausgeben kann.

7. Dosiersystem nach einem der Ansprüche 1 bis 6,
**gekennzeichnet durch**
eine, insbesondere von der Datenverarbeitungsanlage (3) umfasste, Programmiereinheit (22) zur Definition von Arbeitsroutinen und/oder zur Definition von Geräteparametern einer Parametereinheit (23) der Handdosiervorrichtung (2), wobei die Programmiereinheit (22) dazu eingerichtet ist auf der Grundlage von definierten Arbeitsroutinen und/oder Geräteparametern Soll-Betriebsdaten und/oder Soll-Kontextparameterdaten zu erzeugen und zu speichern.

8. Dosiersystem nach einem der Ansprüche 1 bis 7,
**dadurch gekennzeichnet, dass** die Netzwerkzugangseinrichtung (4) von einer Befestigungsvorrichtung (13) zum lösbaren Befestigen von zumindest einer Handdosiervorrichtung (2) umfasst ist.

9. Dosiersystem nach einem der Ansprüche 1 bis 8,
**dadurch gekennzeichnet,**
**dass** die Kontextparametersensoreinheit (8) zur Duplikation oder zur Erfassung eines Messwerts oder eines Ausgangssignals einer labortechnischen Flüssigkeits-Messvorrichtung (24) eingerichtet ist.

10. Verfahren zum Betrieb eines elektronischen Labor-Dosiersystems für Flüssigkeiten mit
zumindest einer elektrischen Handdosiervorrichtung (2), die zumindest eine Betriebssensoreinheit (5) aufweist, die den Betrieb der Handdosiervorrichtung (2) erfasst und diesbezügliche Betriebsdaten erzeugt und insbesondere in einer Speichereinrichtung (6) der Handdosiervorrichtung (2) speichert, wobei die Handdosiervorrichtung (2) ein Schnittstellenmodul (7) aufweist, mit dem die Handdosiervorrichtung (2), insbesondere zur Übertragung der Betriebsdaten, über eine datentechnische Verbindung mit einer externen Netzwerkzugangseinrichtung(4) verbunden wird, wobei die Netzwerkzugangseinrichtung (4) mit einer externen Datenverarbeitungsanlage (3) datentechnisch verbindbar ist, wobei die Datenverarbeitungsanlage (3) datentechnisch mit zumindest einer Kontextparametersensoreinheit (8) verbindbar ist, die den Betrieb der Handdosiervorrichtung (2) zumindest mittelbar erfasst und/oder Betriebsumweltbedingungen der Handdosiervorrichtung (2) erfasst und daraus Kontextparameterdaten erzeugt, wobei das Dosiersystem (1), insbesondere die Datenverarbeitungsanlage (3), eine Vergleichseinheit (9) aufweist, welche zumindest Betriebsdaten der Betriebssensoreinheit (5) mit Soll-Betriebsdaten und/oder Kontextparameterdaten der Kontextparametersensoreinheit (8) mit Soll-Kontextparameterdaten vergleicht und ein Vergleichsergebnis, insbesondere eine im Rahmen des Vergleichs identifizierte Abweichung der Daten von den Solldaten, erzeugt,
**dadurch gekennzeichnet**
**dass** das Dosiersystem (1) eine Dokumentationseinheit (10) umfasst, welche Protokolldatensätze betreffend den Betrieb der zumindest einen Handdosiervorrichtung (2) umfassend zumindest die Betriebsdaten und die Kontextparameterdaten, erzeugt und speichert, wobei die Dokumentationseinheit (10) derart mit der Vergleichseinheit (9) verbunden ist, das der Dokumentationseinheit (10) die Vergleichsergebnisse der Vergleichseinheit (9) übermittelt werden und die Dokumentationseinheit (10) die Vergleichsergebnisse als Teil der Protokolldatensätze den jeweiligen Betriebsdaten und Kontextparameterdaten zugeordnet, speichert, insbesondere nach Ende eines Betriebs der Handdosiervorrichtung (2) ergänzt und die Speicherung und Katalogisierung von Protokolldatensätzen mittels einer Dokumentationsspeichereinrichtung (18) erolgt.

11. Verfahren nach Anspruch 10,
**gekennzeichnet durch**
die Ausgabe von Benutzeranweisungen und/oder Benutzerhinweisen mittels zumindest einer, insbesondere von der Handdosiervorrichtung (2) umfassten, Ausgabeeinheit (17), wobei die Ausgabeeinheit (17) derart mit der Vergleichseinheit (9) verbunden ist, dass Vergleichsergebnisse, insbesondere eine im Rahmen des Vergleichs identifizierte Abweichung der Daten von den Solldaten, unmittelbar ausgegeben werden.

12. Verfahren nach Anspruch 10 oder 11,
**gekennzeichnet durch**
die Erfassung statischer und/oder dynamischer Kontextparameter über zumindest eine Kontextparametersensoreinheit (8).

13. Verfahren nach einem der Ansprüche 10 bis 12,
**gekennzeichnet durch**
die Klassifikation des erfassten Betriebs der Handdosiervorrichtung (2) und/oder der erfassten Betriebsumweltbedingungen mit zumindest einer mit der Kontextparametersensoreinheit (8) verbundenen Klassifizierungseinheit (16), wobei anhand der Klassifizierung ein Kontextparameter abgeleitet und/oder Kontextparameterdaten erzeugt werden.

14. Verfahren nach einem der Ansprüche 10 bis 13,
**gekennzeichnet durch**
die Umwandlung von Protokolldatensätzen in ein Datenformat eines elektronischen Laborbuchs oder zur Einbettung in ein Labor-Informations-Management-System mittels einer Umwandlungseinrichtung (19).

15. Verfahren nach Anspruch 10 bis 14,
**gekennzeichnet durch**
Auswertung von Protokolldatensätzen und/oder Vergleich von Protokolldatensätzen mittels einer Auswerteeinheit (20), welche mit der Dokumentationsspeichereinrichtung (18) verbunden ist und Ausgabe der Ergebnisse der Auswertung, insbesondere zur Weiterverarbeitung seitens des Dosiersystems (1), über eine Ausgabeschnittstelle (21).

16. Verfahren nach einem der Ansprüche 10 bis 15,
**gekennzeichnet durch**
die Definition von Arbeitsroutinen und/oder die Definition von Geräteparametern einer Parametereinheit (23) der Handdosiervorrichtung (2) mit einer, insbesondere von der Datenverarbeitungsanlage (3) umfassten, Programmiereinheit (22), wobei die Programmiereinheit (22) auf der Grundlage von definierten Arbeitsroutinen oder Geräteparametern Soll-Betriebsdaten und/oder Soll-Kontextparameterdaten erzeugt und/oder speichert.

17. Verfahren nach einem der Ansprüche 10 bis 16,
**gekennzeichnet durch**
Duplikation oder die Erfassung eines Messwerts oder eines Ausgangssignals einer labortechnischen Flüssigkeits-Messvorrichtung (24) mit der Kontextparametersensoreinheit (8).

## Claims

1. An electronic laboratory metering system for liquids comprising
at least one electrical handheld metering device (2) having at least one operation sensor unit (5) detecting the operation of the handheld metering device (2) and generating corresponding operating data and, in particular, storing said operating data in a storage device (6) of the handheld metering device (2), the handheld metering device (2) having an interface module (7) by means of which the handheld metering device (2) can be connected to an external network access device (4) via a data connection, in particular for transmitting the operating data, the network access device (4) being data-connectable to an external data processing system (3), the data processing system (3) being data-connectable to at least one context parameter sensor unit (8) at least indirectly detecting the operation of the handheld metering device (2) and/or detecting environmental operating conditions of the handheld metering device (2) and generating context parameter data therefrom, the metering system (1), in particular the data processing system (3), having a comparison unit (9) configured at least to compare operating data of the operation sensor unit (5) with target operating data and/or to compare context parameter data of the context parameter sensor unit (8) with target context parameter data and to generate a comparison result, in particular a deviation of the data from the target data identified in the course of the comparison,
**characterized in that**
the metering system (1) comprises a documentation unit (10) which is configured to generate and to store protocol data sets relating to the operation of the at least one handheld metering device (2) comprising at least the operating data and the context parameter data, the documentation unit (10) being connected to the comparison unit (9) in such a manner that the comparison results of the comparison unit (9) can be transmitted to the documentation unit (10) and the documentation unit (10) is configured to store the comparison results as part of the protocol data sets in a manner assigned to the corresponding operating data and context parameter data, in particular to add to said comparison results after an operation of the handheld metering device (2) has ended, further comprising a documentation storage device (18) which is configured to store and to catalog protocol data sets.

2. The metering system according to claim 1,
**characterized by**
at least one output unit (17), in particular comprised by the handheld metering device (2), for outputting user instructions and/or user information, the output unit (17) being connected to the comparison unit (9) in such a manner that comparison results, in particular a deviation of the data from the target data identified in the course of the comparison, are directly outputted.

3. The metering system according to claim 1 or 2,
**characterized in that**
at least one context parameter sensor unit (8) is configured to detect static and/or dynamic context parameters.

4. The metering system according to any one of claims 1 to 3,
**characterized in that**
at least one context parameter sensor unit (8) is connected to a classification unit (16) which is configured to classify the detected operation of the handheld metering device (2) and/or the detected environmental operating conditions, wherein a context parameter can be derived from the classification and/or context parameter data can be generated by means of the classification.

5. The metering system according to any one of claims 1 to 4,
**characterized by**
a conversion device (19) which is configured to convert protocol data sets into a data format of an electronic laboratory journal or to embed the protocol data sets into laboratory information management systems.

6. The metering system according to claim 4 or 5,
**characterized by**
at least one evaluation unit (20) which is connected to the documentation storage device (18) and which is configured to evaluate protocol data sets and/or to compare protocol data sets and which can output results from the evaluation via an output interface (21), in particular for further processing by the metering system.

7. The metering system according to any one of claims 1 to 6,
**characterized by**
a programming unit (22), in particular comprised by the data processing system (3), for defining work routines and/or for defining device parameters of a parameter unit (23) of the handheld metering device (2), the programming unit (22) being configured to generate and to store target operating data and/or target context parameter data based on defined work routines and/or device parameters.

8. The metering system according to any one of claims 1 to 7,
**characterized in that**
the network access device (4) is comprised by a fastening device (13) for the detachable fastening of at least one handheld metering device (2).

9. The metering system according to any one of claims 1 to 8,
**characterized in that**
the context parameter sensor unit (8) is configured to duplicate or to detect a measured value or an output signal of a laboratory liquid measuring device (24).

10. A method for operating an electronic laboratory metering system for liquids, the system comprising
at least one electrical handheld metering device (2) having at least one operation sensor unit (5) detecting the operation of the handheld metering device (2) and generating corresponding operating data and, in particular, storing said operating data in a storage device (6) of the handheld metering device (2), the handheld metering device (2) having an interface module (7) by means of which the handheld metering device (2) is connected to an external network access device (4) via a data connection, in particular for transmitting the operating data, the network access device (4) being data-connectable to an external data processing system (3), the data processing system (3) being data-connectable to at least one context parameter sensor unit (8) at least indirectly detecting the operation of the handheld metering device (2) and/or detecting environmental operating conditions of the handheld metering device (2) and generating context parameter data therefrom, the metering system (1), in particular the data processing system (3), having a comparison unit (9) at least comparing operating data of the operation sensor unit (5) with target operating data and/or comparing context parameter data of the context parameter sensor unit (8) with target context parameter data and generating a comparison result, in particular a deviation of the data from the target data identified in the course of the comparison,
**characterized in that**
the metering system (1) comprises a documentation unit (10) which generates and stores protocol data sets relating to the operation of the at least one handheld metering device (2) comprising at least the operating data and the context parameter data, the documentation unit (10) being connected to the comparison unit (9) in such a manner that the comparison results of the comparison unit (9) are transmitted to the documentation unit (10) and the documentation unit (10) stores the comparison results as part of the protocol data sets in a manner assigned to the corresponding operating data and/or context parameter data, in particular adds to said comparison results after an operation of the handheld metering device (2) has ended and the storage and/or cataloging of protocol data sets by means of a documentation storage device (18) has taken place.

11. The method according to claim 10,
**characterized by**
the output of user instructions and/or user information by means of at least one output unit (17), in particular comprised by the handheld metering device (2), the output unit (17) being connected to the comparison unit (9) in such a manner that comparison results, in particular a deviation of the data from the target data identified in the course of the comparison, are directly outputted.

12. The method according to claim 10 or 11,
**characterized by**
the detection of static and/or dynamic context parameters via at least one context parameter sensor unit (8).

13. The method according to any one of claims 10 to 12,
**characterized by**
the classification of the detected operation of the handheld metering device (2) and/or the detected environmental operating conditions by means of at least one classification unit (16) which is connected to the context parameter sensor unit (8), a context parameter being derived from the classification and/or context parameter data being generated by means of the classification.

14. The method according to any one of claims 10 to 13,
**characterized by**
the conversion of protocol data sets into a data format of an electronic laboratory journal or the embedding into a laboratory information management system by means of a conversion device (19).

15. The method according to claim 10 or 14,
**characterized by**
the evaluation of protocol data sets and/or the comparison of protocol data sets by means of an evaluation unit (20) which is connected to the documentation storage device (18) and the output of the results from the evaluation via an output interface (21), in particular for further processing by the metering system (1).

16. The method according to any one of claims 10 to 15,
**characterized by**
the definition of work routines and/or the definition of device parameters of a parameter unit (23) of the handheld metering device (2) by means of a programming unit (22), in particular comprised by the data processing system (3), the programming unit (22) generating and/or storing target operating data and/or target context parameter data based on defined work routines or device parameters.

17. The method according to any one of claims 10 to 16,
**characterized by**
the duplication or the detection of a measured value or an output signal of a laboratory liquid measuring device (24) by means of the context parameter sensor unit (8).

## Revendications

1. Système de dosage de laboratoire électronique pour des liquides, ledit système de dosage comprenant
au moins un dispositif (2) de dosage à main électrique ayant au moins une unité (5) de capteur de fonctionnement détectant le fonctionnement du dispositif (2) de dosage à main et générant des données de fonctionnement se référant à celui-ci et, notamment, mémorisant ces données dans un moyen de mémoire (6) du dispositif (2) de dosage à main, le dispositif (2) de dosage à main ayant un module d'interface (7) au moyen duquel le dispositif (2) de dosage à main peut être relié à un moyen (4) d'accès au réseau externe via une liaison utilisant la technologie informatique, notamment afin de transmettre les données de fonctionnement, le moyen (4) d'accès au réseau pouvant être relié à une installation (3) de traitement de données externe en utilisant la technologie informatique, l'installation (3) de traitement de données pouvant être reliée à au moins une unité (8) de capteur de paramètres de contexte en utilisant la technologie informatique, l'unité (8) de capteur de paramètres de contexte détectant au moins indirectement le fonctionnement du dispositif (2) de dosage à main et/ou détectant des conditions environnementales du fonctionnement du dispositif (2) de dosage à main et générant des données de paramètres de contexte à partir de cela, le système de dosage (1), notamment l'installation (3) de traitement de données, ayant une unité de comparaison (9) configurée au moins pour comparer des données de fonctionnement de l'unité (5) de capteur de fonctionnement à des données de fonctionnement de consigne et/ou des données de paramètres de contexte de l'unité (8) de capteur de paramètres de contexte à des données de paramètres de contexte de consigne et pour générer un résultat de comparaison, notamment un écart des données, qui a été identifié dans le cadre de la comparaison, des données de consigne,
**caractérisé en ce que**
le système de dosage (1) comprend une unité de documentation (10) qui est configurée pour générer et mémoriser des ensembles de données de protocole relatifs au fonctionnement de l'au moins un dispositif (2) de dosage à main comprenant au moins les données de fonctionnement et les données de paramètres de contexte, dans lequel l'unité de documentation (10) est reliée à l'unité de comparaison (9) de telle manière que les résultats de comparaison de l'unité de comparaison (9) peuvent être transmis à l'unité de documentation (10), et dans lequel l'unité de documentation (10) est configurée pour mémoriser les résultats de comparaison comme partie des ensembles de données de protocole de manière assignée aux données de fonctionnement et/ou données de paramètres de contexte respectives, notamment pour les ajouter à la fin d'un fonctionnement du dispositif (2) de dosage à main, comprenant en outre un moyen (18) de mémoire de documentation qui est configuré pour mémoriser et/ou cataloguer des ensembles de données de protocole.

2. Système de dosage selon la revendication 1,
**caractérisé par**
au moins une unité de sortie (17), qui est notamment comprise par le dispositif (2) de dosage à main, pour la sortie d'instructions d'utilisateur et/ou d'informations d'utilisateur, l'unité de sortie (17) étant reliée à l'unité de comparaison (9) de telle manière que des résultats de comparaison, notamment un écart des données, qui a été identifié dans le cadre de la comparaison, des données de consigne, sont directement délivrés.

3. Système de dosage selon la revendication 1 ou la revendication 2,
**caractérisé en ce**
**qu'**au moins une unité (8) de capteur de paramètres de contexte est configurée pour détecter des paramètres de contexte statiques et/ou dynamiques.

4. Système de dosage selon l'une quelconque des revendications 1 à 3,
**caractérisé en ce**
**qu'**au moins une unité (8) de capteur de paramètres de contexte est reliée à une unité de classification (16) qui est configurée pour classer le fonctionnement détecté du dispositif (2) de dosage à main et/ou les conditions environnementales du fonctionnement détectées, un paramètre de contexte pouvant être déduit de et/ou des données de paramètres de contexte pouvant être générées au moyen de la classification.

5. Système de dosage selon l'une quelconque des revendications 1 à 4,
**caractérisé par**
un moyen de conversion (19) qui est configuré pour convertir des ensembles de données de protocole en un format de données d'un cahier de laboratoire électronique ou pour intégrer les ensembles de données de protocole dans un système de gestion de l'information du laboratoire.

6. Système de dosage selon la revendication 4 ou la revendication 5,
**caractérisé par**
au moins une unité d'évaluation (20) qui est reliée au moyen (18) de mémoire de documentation et qui est configurée pour évaluer des ensembles de données de protocole et/ou pour comparer des ensembles de données de protocole et qui peut délivrer des résultats de l'évaluation via une interface de sortie (21), notamment pour le traitement ultérieur par le système de dosage.

7. Système de dosage selon l'une quelconque des revendications 1 à 6,
**caractérisé par**
une unité de programmation (22), qui est notamment comprise par l'installation (3) de traitement de données, pour la définition de routines de travail et/ou pour la définition de paramètres d'appareil d'une unité de paramètres (23) du dispositif (2) de dosage à main, l'unité de programmation (22) étant configurée pour générer et mémoriser des données de fonctionnement de consigne et/ou des données de paramètres de contexte de consigne sur la base de routines de travail et/ou paramètres d'appareil défini(e)s.

8. Système de dosage selon l'une quelconque des revendications 1 à 7,
**caractérisé en ce que**
le moyen d'accès de réseau (4) est compris par un dispositif de fixation (13) pour la fixation amovible d'au moins un dispositif (2) de dosage à main.

9. Système de dosage selon l'une quelconque des revendications 1 à 8,
**caractérisé en ce que**
l'unité (8) de capteur de paramètres de contexte est configurée pour dupliquer ou pour détecter une valeur de mesure ou un signal de sortie d'un dispositif (24) de mesure de liquides utilisant la technologie de laboratoire.

10. Procédé de fonctionnement d'un système de dosage de laboratoire électronique pour des liquides, ledit système de dosage comprenant
au moins un dispositif (2) de dosage à main électrique ayant au moins une unité (5) de capteur de fonctionnement détectant le fonctionnement du dispositif (2) de dosage à main et générant des données de fonctionnement se référant à celui-ci et, notamment, mémorisant ces données dans un moyen de mémoire (6) du dispositif (2) de dosage à main, le dispositif (2) de dosage à main ayant un module d'interface (7) au moyen duquel le dispositif (2) de dosage à main est relié à un moyen (4) d'accès au réseau externe via une liaison utilisant la technologie informatique, notamment afin de transmettre les données de fonctionnement, le moyen (4) d'accès au réseau pouvant être relié à une installation (3) de traitement de données externe en utilisant la technologie informatique, l'installation (3) de traitement de données pouvant être reliée à au moins une unité (8) de capteur de paramètres de contexte en utilisant la technologie informatique, l'unité (8) de capteur de paramètres de contexte détectant au moins indirectement le fonctionnement du dispositif (2) de dosage à main et/ou détectant des conditions environnementales du fonctionnement du dispositif (2) de dosage à main et générant des données de paramètres de contexte à partir de cela, le système de dosage (1), notamment l'installation (3) de traitement de données, ayant une unité de comparaison (9) comparant au moins des données de fonctionnement de l'unité (5) de capteur de fonctionnement à des données de fonctionnement de consigne et/ou des données de paramètres de contexte de l'unité (8) de capteur de paramètres de contexte à des données de paramètres de contexte de consigne et générant un résultat de comparaison, notamment un écart des données, qui a été identifié dans le cadre de la comparaison, des données de consigne,
**caractérisé en ce que**
le système de dosage (1) comprend une unité de documentation (10) qui génère et mémorise des ensembles de données de protocole relatifs au fonctionnement de l'au moins un dispositif (2) de dosage à main comprenant au moins les données de fonctionnement et les données de paramètres de contexte, dans lequel l'unité de documentation (10) est reliée à l'unité de comparaison (9) de telle manière que les résultats de comparaison de l'unité de comparaison (9) sont transmis à l'unité de documentation (10), et dans lequel l'unité de documentation (10) mémorise les résultats de comparaison comme partie des ensembles de données de protocole de manière assignée aux données de fonctionnement et données de paramètres de contexte respectives, notamment les ajoute à la fin d'un fonctionnement du dispositif (2) de dosage à main et la mise en mémoire et le catalogage des ensembles de données de protocole au moyen d'un moyen (18) de mémoire de documentation ont lieu.

11. Procédé selon la revendication 10,
**caractérisé par**
la sortie d'instructions d'utilisateur et/ou d'informations d'utilisateur au moyen d'au moins une unité de sortie (17) qui est notamment comprise par le dispositif (2) de dosage à main, l'unité de sortie (17) étant reliée à l'unité de comparaison (9) de telle manière que des résultats de comparaison, notamment un écart des données, qui a été identifié dans le cadre de la comparaison, des données de consigne, sont directement délivrés.

12. Procédé selon la revendication 10 ou la revendication 11,
**caractérisé par**
la détection des paramètres de contexte statiques et/ou dynamiques par au moins une unité (8) de capteur de paramètres de contexte.

13. Procédé selon l'une quelconque des revendications 10 à 12,
**caractérisé par**
la classification du fonctionnement détecté du dispositif (2) de dosage à main et/ou des conditions environnementales du fonctionnement détectées au moyen d'au moins une unité de classification (16) qui est reliée à l'unité (8) de capteur de paramètres de contexte, un paramètre de contexte étant déduit de et/ou des données de paramètres de contexte étant générées au moyen de la classification.

14. Procédé selon l'une quelconque des revendications 10 à 13,
**caractérisé par**
la conversion des ensembles de données de protocole en un format de données d'un cahier de laboratoire électronique ou l'intégration dans un système de gestion de l'information du laboratoire au moyen d'un dispositif de conversion (19).

15. Procédé selon la revendication 10 ou la revendication 14,
**caractérisé par**
l'évaluation des ensembles de données de protocole et/ou la comparaison des ensembles de données de protocole au moyen d'une unité d'évaluation (20) qui est reliée au moyen (18) de mémoire de documentation et par la sortie des résultats de l'évaluation via une interface de sortie (21), notamment pour le traitement ultérieur par le système de dosage (1).

16. Procédé selon l'une quelconque des revendications 10 à 15,
**caractérisé par**
la définition de routines de travail et/ou la définition de paramètres d'appareil d'une unité de paramètres (23) du dispositif (2) de dosage à main au moyen d'une unité de programmation (22), qui est notamment comprise par l'installation (3) de traitement de données, l'unité de programmation (22) générant et/ou mémorisant des données de fonctionnement de consigne et/ou des données de paramètres de contexte de consigne sur la base de routines de travail ou paramètres d'appareil défini(e)s.

17. Procédé selon l'une quelconque des revendications 10 à 16,
**caractérisé par**
la duplication ou la détection d'une valeur de mesure ou d'un signal de sortie d'un dispositif (24) de mesure de liquides, qui utilise la technologie de laboratoire, au moyen de l'unité (8) de capteur de paramètres de contexte.
